# EUROPEAN PATENT SPECIFICATION

(11) **EP 2 193 209 B1**
(45) Date of publication and mention of the grant of the patent: **12.11.2014**
(21) Application number: 08831777.1
(22) Date of filing: 16.09.2008
(51) Int. Cl.: C12Q 1/68

(54) **METHOD FOR THE ANALYSIS OF BREAST CANCER DISORDERS**
VERFAHREN ZUR ANALYSE VON BRUSTKREBSERKRANKUNGEN
PROCÉDÉ D'ANALYSE DES TROUBLES LIÉS AU CANCER DU SEIN

(30) Priority: 17.09.2007 US 972843 P
(43) Date of publication of application: 09.06.2010
(73) Proprietor: Koninklijke Philips N.V., 5656 AE Eindhoven (NL); Cold Spring Harbor Laboratory, New York, NY 11724 (US)
(72) Inventor: KAMALAKARAN, Sitharthan, NL-5656 AE Eindhoven (NL); LUCITO, Robert, NL-5656 AE Eindhoven (NL); HICKS, James Bruce, NL-5656 AE Eindhoven (NL); ZHAO, Xiaoyue, NL-5656 AE Eindhoven (NL); KENDALL, Jude, NL-5656 AE Eindhoven (NL)
(74) Representative: Kroeze, Johannes Antonius
(86) International application number: PCT/IB2008/053745
(87) International publication number: WO 2009/037635

(56) References cited:
- WO-A-02/18632
- WO-A-2005/059172
- WO-A-2005/123945
- WO-A-2006/008128
- WO-A-2007/019670
- MIYAMOTO KAZUAKI ET AL: "Diagnostic and therapeutic applications of epigenetics." JAPANESE JOURNAL OF CLINICAL ONCOLOGY JUN 2005, vol. 35, no. 6, June 2005 (2005-06), pages 293-301, XP007907854 ISSN: 0368-2811
- YANG X ET AL: "DNA methylation in breast cancer." ENDOCRINE-RELATED CANCER JUN 2001, vol. 8, no. 2, June 2001 (2001-06), pages 115-127, XP007907855 ISSN: 1351-0088

### SEQUENCE LISTING

<110> Philips Intellectual Property & Standards GmbH
<120> METHOD FOR THE ANALYSIS OF BREAST CANCER DISORDERS
<130> PH009058 EP-1
<160> 100
<170> PatentIn version 3.4
<210> 1
   <211> 582
   <212> DNA
   <213> Homo sapiens
<400> 1
   gggacttgca cgggtggcgc gcctagccca gagcccgctt gtagtgagcg ctcagtaggg     60
   cgggaaagct gctgccgcta ctgcggcctt ctgagaagac gcccattctc tgatgtcagg    120
   actgcatcgc cgagtcctga ttgcaaatag cgaaatatat aattaaggag agttcggggg    180
   cgtatcctgc aatgggatgc cgagttcgat aaagttgcag gatgctggga aagtggattg    240
   atttgctgtt gctaaatgaa tggaaacagc gttagagctt ctgtccacga tccatcagtc    300
   tatcgagatt gctgattgcc tatgacttgg acggttgatg gttttcaggc ccactgagag    360
   cagccagcgt tggtataacc gcagggagcc ctcctgggag gttgaacctg cagagcgcaa    420
   atgtgcccag gttgatttgg ggcctagtgg tccattttca cagctccact gcctatctgc    480
   ataaccagga gccctcattc agatgaacgc caaatgggcc agagggagga gaatcaaatg    540
   agcaactttt aaaagatatt aagacatagg cacctaactc cg                       582
<210> 2
   <211> 659
   <212> DNA
   <213> Homo sapiens
<400> 2
   gatacactcc tattatatgt gcctatcatc ctgaggagta atttgattca ggtgttctgg     60
   aagtcatgct gtgggctgtg tctgttgaat tcccagcgat gccaggggac acaccctgtg    120
   actccttcct gaattgagtg ctgatatttg attggcttat cgcgcacctg atgagtgggt    180
   ggggtgttcg cggttggtgg gggtgactta cagaagggct gatgcggcca gagagctcgt    240
   catttgaaga ctctctcgga agggatagcg tctttctgca acctgcggtc ccagcagaca    300
   aaccttgtga tcctcgttcc agtcgacatg gaggacgact cactctactt gagaggtgag    360
   tggcagttca accacttttc aaaactcaca tcttctcggc ccgatgcagc ttttgctgaa    420
   atccagcgga cttctctccc tgagaagtca ccactctcat gtgagacccg tgtcgacctc    480
   tgtgatgatt tggctcctgt ggcaagacag cttgctccca gggagaagct tcctctgagt    540
   agcaggagac ctgctgcggt gggggctggg ctccagaata tgggaaatac ctgctacgtg    600
   aacgcttcct tgcagtgcct gacatacaca ccgccccttg ccaactacat gctgtcccg     659
<210> 3
   <211> 495
   <212> DNA
   <213> Homo sapiens
<400> 3
   ggacacttaa ttcttcctcc aatccctggt ctctgccgcg tgggctagat ctactgcaag      60
   tgctgggcat ggggaaagga agagagaagt taaaggtgaa atggcttctg ctctcggcct     120
   ctccgtgagc gctctcggcc ctcctgtgga gatcgccacg tcctatcaca cgcgtcccta     180
   aaccttgtca ctcgagcatt cttgtctccc aaactctgat ggtccgcagt gggtactggg     240
   ctccctgggc cctgggagga agacggagga gtttgggtga aagatcagac ggtgcgcagc     300
   cctgaggctt ctgagggcag agggtgcgct tccttgccct cggggcgggg aagccgtgaa     360
   cgccgaggcc attgtaaggc tgggtggtgc ggagcgcggg aagggggctg ggatttgaat     420
   gggagcctgt gattggccga tccctggact gacgtcactt ccccgcgggg cgattagcct     480
   gcgagaggag ggccg                                                      495
<210> 4
   <211> 150
   <212> DNA
   <213> Homo sapiens
<400> 4
   gcgcgctaca gactcccgag aacagccctg gctgtcagcg agcaccagcc gcttcctgtc      60
   cccatcgcgg agactggagg ggcgcaccac ggccatggag ccagaggcgc ttcaggaggc     120
   aagagaagtc cccgcgcgct ccgcagcccg                                      150
<210> 5
   <211> 360
   <212> DNA
   <213> Homo sapiens
<400> 5
   gtatgggtca cgtgacccag ctgtgctgcc ttacacgctt gcctccatgc ctgtgcctcg      60
   gccgtccccg cgccagacac ccttgacctg gtccacccag cgcgtggctt ttgtcactgc     120
   cctgcgctcc tcactgttcc tgagtctccc gacgggctga aacagggttg ttggtttgca     180
   gggcctgcac agggccctca gcactcggat aaagaaagga aagacggaca ccagcctgcg     240
   cccacagcga gaaccccaga gtgggagcca gcacggagcc aaggctactg cggggccgct     300
   cgcctgcatc agcccctgct actgccaggc tggagccaag gtacggcgcg cacacacccg     360
<210> 6
   <211> 325
   <212> DNA
   <213> Homo sapiens
<400> 6
   gaacgcccct ggccaggctc catctacgcg ctgtcagacc ctcccgccgt ctgaagaagg 60
   cttttactct tcagcctatt ccagtggcag agaagctaag gctacaaagg cgaacgcgaa 120
   cagtcagatc tgacttcgaa ttccgctgtc attgctgcca ggcgcaccac gaggacgcgc 180
   ggtgaccgcc accatggcat tcggctgcca aaggtttcca tcgacctctt tcccatcacc 240
   agcatcgcag cgggaaagaa tgtgcctggc gcccttctgg gcactgggca tggggtggtg 300
   aacaaagtcc tccagaaata aaccg 325
<210> 7
   <211> 234
   <212> DNA
   <213> Homo sapiens
<400> 7
   gggcggctgc caacctcacc tcttagtcaa ccagctgttt gacctgtgaa ttaggcccgt 60
   aaaagacttc gtttcttgag aaaataagat tggagtccgt cgtaggaaac tggaaccgaa 120
   ttcagagaaa gcgattcacc gaaaaggaaa tgaccaattg cctgagtttc cgaaatggca 180
   gaggactggc cctgctgtgc gcgctcctgg ggacgctgtg cgaaacagga tccg 234
<210> 8
   <211> 392
   <212> DNA
   <213> Homo sapiens
<400> 8
   ggtgcctcag acatcaagcg aggctactac tggcaacaaa atcccccact tccaaaatcc 60
   ctttttctgg gaaaggcaga atccgcctgc agcagccaat tgggcaggcg ccaagaaagt 120
   agtgaagggt cagaggggac aggaggccac ccgcagcgcc caccctggat agggataggt 180
   tcgagaggtt gctactgagc tgcaaaataa ccccgtcttt ccttcccgca tatggtcttg 240
   tatctgacag tccctgtgtc tcacagcggt aatcgtgtgt tgcatcgatg cgtttgtggt 300
   caccacgaag atgaggacca acttgaaaag attcctggga gtcgaagttg aaaggaagct 360
   ttcccccgcc aaggacgcct accccgaaac cg 392
<210> 9
   <211> 336
   <212> DNA
   <213> Homo sapiens
<400> 9
   ggccgtggct ctttcagcac cgaatcctag ccactagaca accatgcaga tgcggaaagc      60
   tgctttctct cccttcttcg acctgaagcg acactttcct gtgctctagg aggacttggg     120
   tcttgtgaga gtcgcccttt gctcctggag tcgtctcaca aggccgttca ctccctgctt     180
   tcttcaaaaa aagaacctgc aggcgacaca ccaaggactc cacgagggag tcctgagtac     240
   tggagcgagt tgcggccacg cggccgcagc tcaccactgg cctagagatg ccctttgcga     300
   ggcggcagca actgacaaga tggtcgcggg tcgccg                               336
<210> 10
   <211> 495
   <212> DNA
   <213> Homo sapiens
<400> 10
   gtgcgccagg gaaggagctt gatggtgtgt acggctacgt agtgaacgtg gaggtgtgcg      60
   cagcggagtc agaggggcta gatccccagc atttcccacg gggtcccacc tagcgcgtgg     120
   atgggagcaa cttcatcact ccaccgaggg cagggaaaga ggagtaagca ttggagctca     180
   aagaatgagc aatttttcaa agctttctcc gcaggcctgg gcagacacac acgcacacac     240
   acacacaccg cacccccaaa actcaaaaga atgttatggt tgacctagtc tgtgcgtgtg    300
   ctgtatgaga gtatttgtga gcttatgtgg gtgcccactt ccctttctat tttgctcctg     360
   ggtggaaatt tggtcccaga aattattcca ggaacagaag gagcgagctg gagggccgca     420
   ggagaagggg gcgctacact tccaaggagc ggactttgga agcccccacc tccaccacag     480
   cactattggt ggccg                                                      495
<210> 11
   <211> 289
   <212> DNA
   <213> Homo sapiens
<400> 11
   gatttggggt ggcggttaat gctcctcttc tggtgcctcc cccgctgggg ccctgcattc      60
   cattagcccc gcgctggggt agagatgcca agtttagggg ctggagagga caaggggaag     120
   gacgagagaa ctcttgcacc gacggatgta gttccaagcg acccgaaatg aggaaaaccc     180
   agaaggggcg gacggagggc aaagtcggcc caggacgatc ttcaggcgca ccattctctc     240
   gccctggagg gacagggaag ggcggaagcg gagggtggct gtccagccg                  289
<210> 12
   <211> 342
   <212> DNA
   <213> Homo sapiens
<400> 12
   ggccgtgacg ctttcagcac cgaatcctag ccactagaca accatgcaga tgcggaaagc      60
   tgctttctct cccttcttcg acctgaagcg acactttcct gtgctctagg aggacttggg     120
   tcttgtgaga gtcgctcttt gctcctggag tcgtctcaca aggccgttca ctccctgctt     180
   tcttcaaaaa aagaacctgc aggcgacaca ccaagggctc cacgagggag tcctgagtac     240
   tggagcgagt tgcggccacg cggccgcagc tcaccactgg cctagagatg ccctttgcga     300
   ggcggcagca actgacaaga tggtcgcggg tcgccgcgtc cg                        342
<210> 13
   <211> 463
   <212> DNA
   <213> Homo sapiens
<400> 13
   gtgcctgtgt tgttgggcaa agccagggga aaggtcaggt cgccttggga tctggcctag      60
   cactgagtgc agaggaagag aagcttctga gatcgagagg cagcggctac aggaggagga     120
   agaaagcaag gcggggaaaa ccaaagttca ggagagcatt tgcaggaggg cgctgcgggg     180
   tagggtctga gtcggagccc cgaatcggac tcgagtcctg tcgcgtgggg acggagcgtg     240
   cagaggccca tgcgagggac agacacaaag gccctgggag ctgcaggtca gaccactgga     300
   cagcgcggcc gcggacctaa cgcccctgta aggggtgctc acgcttgggg ggaactctcc     360
   gaaagagagg accactgaaa gccaccctgg cagcaggggc ggaacagaca gacgtgggtc     420
   tggccgtcgc cagaactgcc tgcggaccag ccccgcgctg ccg                       463
<210> 14
   <211> 501
   <212> DNA
   <213> Homo sapiens
<400> 14
   gcggggacca cgtggagggg cctggtcagc aagtggcaag gacgcggcgc aaagccaggc 60
   tttctaatgt acaatccgcg gctctttcga tactgaacac ccacctcttc aacaaattcg 120
   agcgcctggc aggccctgca ccaagcctag ggtgatggcg acccaggcgg gtttttactc 180
   tcaaggagct caattctagg gcagggagaa gtaaaattaa aaaaaaaaaa atcataacaa 240
   actgaaaaac acataaataa gccacgtctc tcctcacccc tagcacttaa tcacaaaggc 300
   ctgtagagag tcccgacgag aacttctgag caggccccgc tgtcagtccc tgaggacagc 360
   atgcaaggga ggttgacgtc cctggacgaa gaaagtgctg gagaaactgg cgaccgtccc 420
   accttcccta aggcgaaggc tctgaattcc gaagaccgtt actcggcgcg gaacgcgggc 480
   accgccgacg tcaaaacacc g 501
<210> 15
   <211> 388
   <212> DNA
   <213> Homo sapiens
<400> 15
   gtactgcgta aggggtgtgc gtggctttgc agttcgccta ggagacaagc tgatgcacgg 60
   cctggaaggt gcagccctca tccctgcgtt ccctccccgc gcggagttcc tgtctcaagg 120
   ccaagacgcg cagcgctgat gctggcccct tcctaggtca gtgcagctgg accaggcaat 180
   cggggtctcc gaaacgtcgg ggggtcagct ggcgccctgc ccaccatcga acgcccttcg 240
   tatcactccg ctcctcattg cgaattggac tgcgcattgg ctgcgaccca aggctgaaca 300
   caagaagctt ggagaggagc tcgggcgttt gaggagaagg aaatatgagg aattgcagga 360
   aggcgctcgc ctgcccgaac tgcctccg 388
<210> 16
   <211> 613
   <212> DNA
   <213> Homo sapiens
<400> 16
   gcgacccgcg ggtgggcggg ctcatgggcg ccctctggtg cttcgctccc tggctttgcg      60
   ctcggagctt tgacttgaga atccatatcc ttcgttcagc acaaagtttc tgagcgccta     120
   ccacgtggag agagctaagt gctaggtgct gtgaagaaac acagcgttta agacaggatc     180
   cctgtcttcc agaagctttt caaataaacg gattgattcc aggaataaat agcttaacct     240
   ttctgagcct caaatctgtc tttggtaaaa tagaataatt atatttcacc ccgtagtttt     300
   gaagataaca tgagaatggg tgtaaaagac ctgttgtcaa tgcgtatctc aaacactaaa     360
   caaatatctt ttaaaaatat cataccctaa gagtctggcg cggtggcgtc tgcctgtaat     420
   tacagagatg gtgaaacctt gtctctataa acacaaacac acacatattt tttgacagtc     480
   tcacgatgtc acccaggctg gggtgcagtg gcacgatctc ggctcactgc aatctctgcc     540
   tcccaggttc atgcagttat cctgcctcag tctctcgagt agctggggtt acaggtgcat     600
   gtcaccacac ccg                                                        613
<210> 17
   <211> 215
   <212> DNA
   <213> Homo sapiens
<400> 17
   ggtccagggg gggcagggtt cgaggcgtgg cctagaggcg ttgtaggcgt cgacccattt      60
   cgtcggctga gagactgggt cttgtgtgga cggtgctatt ctgggcgaaa gggttagctt     120
   atggttgtgg cctcgcattc ctgagtgcgc gatgacggac tcggcgggtg cgtgagagac     180
   cagggccgag gaggggtctg tgcaggcggc ttccg                                215
<210> 18
   <211> 418
   <212> DNA
   <213> Homo sapiens
<400> 18
   ggaacctaca ggcctcgggc cgacccagga agcctccgca ccagaaagct cgaggagccc      60
   ttacccaagt cttgctcgaa gggcaaagca aagagccagc acccctgagt gtcactgaag     120
   ttcctggatg gggtgtgagt gcgcgcgttc cgtccgagac ctcagtctcg cccagctata     180
   gagccgataa agggatgtct tgtgggcgta aggcgcttcg cgcccatctc caaggccgat     240
   gtggtcagga ggtgagggga aatgtccttc tggcagaagc ccgcggtgct gcgacgttga     300
   cccgcctggc ctcaggctca gggcggcggg cagcccaggg cacatgtagt ttcagcagcc     360
   gcgctacgtg ggcgggggac cccaggccac cccacgtgtc cgccctgggc ctcctccg       418
<210> 19
   <211> 483
   <212> DNA
   <213> Homo sapiens
<400> 19
   gatttgcgag cagcttgtca gctgtttttg ccctcccctt cccatccatc cactgcgaat     60
   gagtattagg aattcagaag cgttgtttgc tatttgatcc taagggaaac tactgtacta    120
   aatatattct tctgatacat gttagcacct ctaaagtatc tgcaaaccat caagaggtag    180
   ttgcgaagac tcaaaatcct aaatcacgca ctcatcagta cgcagctgtt gcgaaacagc    240
   tcttaaattg ccactttaaa cccaaagaga gtatgtctct agcctcagct gctacctaat    300
   attgatcaag cactcactac gtgcaagaga caggaactgt ggagaaggct ggggaaaaaa    360
   gcgtgaaaac cacgaaaaca tcagaagcca aaactcaaga acctctacta ggctaactga    420
   ttagtgaaaa cgtcgtgtta acagcaaacg ccttgagtgt agcgctccta ctcctaaccc    480
   ccg                                                                  483
<210> 20
   <211> 345
   <212> DNA
   <213> Homo sapiens
<400> 20
   ggccgccagg gtccctcact cccgagttct caaagggcgg aggggtttgg gaggccgtct     60
   ggttacctat taaccctgcg gctccttctg agcttgccag gagctggaga gagcagagca    120
   gcccagcgcc caagcctgac ccagtgaatg cgatgctccc cgccctcccg cgcaatctca    180
   gaggcgggct tccctcggaa aacggagggt tgaaacccag aaagctcctc ggagcccttc    240
   tggggttcgg gcgatctggg cagagaactc tcgggtggca aggaattaag ggcagcgcga    300
   acgacggtct ctagacaagt atgaagcctc ccctgagtgt gtccg                    345
<210> 21
   <211> 441
   <212> DNA
   <213> Homo sapiens
<400> 21
   ggggccacgt aatgctgagt gctgattggc tgctcttggc tcctcccctc atcccgcttt     60
   tggcccaaga gcgtggtgca gattcacccg cgcgaggtag gcgctctggt gcttgcggag    120
   gacgcttcct tcctcagatg caccgatctt cccgatactg cctttggagc ggctagattg    180
   ctagccttgg ctgctccatt ggcctgcctt gccccttacc tgccgattgc atatgaactc    240
   ttcttctgtc tgtacatcgt tgtcgtcgga gtcgtcgcga tcgtcgtggc gctcgtgtga    300
   tggccttcgt ccgtttagag tagtgtagtt agttaggggc caacgaagaa gaaagaagac    360
   gcgattagtg cagagatgct ggaggtggtc agttactaag ctagagtaag atagcggagc    420
   gaaaagagcc aaacctagcc g                                               441
<210> 22
   <211> 425
   <212> DNA
   <213> Homo sapiens
<400> 22
   ggagggtctg aacggcgggg gcctcctgga gcgccttcag cgcctcgtgt tttctttcct     60
   tgggtccctg ttagcgtcgc cgcatgcaca gcgctccctg tcatttggtc cctgagcttg    120
   ggtgtttttc acatttcatc taaagatgca gacccctgct aagtctagcc agccttcacc    180
   ctgaatcttg aacttaagat gctgcatttt ccacccaccc ccacccctct agtagctgca    240
   gattgacaag cggcttttcc caaccagatg acattttggc tgcaagaatc cgtggcccgt    300
   tcttgcctct gtgcagccaa gctccttcgc tgtctttctc aaactgcagt cctgagttca    360
   gcttcgctta gcagtgacca tcccagaggt gggtaatcca acactggatc cactcactgc    420
   aaccg                                                                 425
<210> 23
   <211> 771
   <212> DNA
   <213> Homo sapiens
<400> 23
   gcgttccccc cagacacgcc catagccacg cgttcgggcc tctttcggtc atcttttcgc      60
   ctctagtgag cacacgattt aaggatgatt tggctcgtca ttttgctgag gattttgtct     120
   agataatcat tccttggaac gatcggagaa atgcctcttt ccctccctgg ctgccgtcca     180
   cacatcgttg gtggcagact gtgagggaaa tcgttaaaat gactcaaaag caatgtgtaa     240
   cacaagtgat agagcaggga gcttttcccc acagggtttg gagctacttg atattcataa     300
   aataaagcag ctatatacat ctctgaagcc aaaagagcca tgcaggggaa aggggtaatt     360
   cccaaaggac actataattc gtgttagtcc taaacctatt tctcacctgc cattcctact     420
   gatttgttcc ctgtccttag tccaaacctg tctcgtctca tagaaacctt ccgaactcac     480
   ttctgtggtg caagcgcttt tttcccaacc ccaaactcca atgccccacc cttaggaagt     540
   ggaggaagtc aagcagtggt ctgcagattt ccagatgcac tcaaggatga ttaaatttct     600
   cagaacctac aaacctatat ttaattattg aaaacgggcg gggcgtggta gctcatgcct     660
   gtaatcccag cactttggga ggccgaggcg ggtgtatcgg tcagaagttc gagaccagcc     720
   tgatcaacat ggtgaaaccc tgcctctact aaaaatacaa aaattagccc g              771
<210> 24
   <211> 334
   <212> DNA
   <213> Homo sapiens
<400> 24
   gcctctggac catcgtggag gtccctcctg tcttttgagc atctaaatgt ggatctccga      60
   ggacctcggg gcatgagaga tggaggggcc gtgtgctgag tctccctgtc caggctcagg     120
   tgcctctgct gcccttggtt tgcgccagga agtgcttcct ccctggcctg gcctgagaga     180
   gtcacggggt tgtgtagacc taaggctctc aactggggag gacttaaccc cccaggacgt     240
   ttgacaatgt gtggagataa ttgtctcgct gcatgggtgg ggtgggtgct tctggcatct     300
   ggtggggaga ggcctgggat ggtgctaaac cccg                                334
<210> 25
   <211> 409
   <212> DNA
   <213> Homo sapiens
<400> 25
   gagatcacag tggaggcacc agcgatccga gagtcctatg acgaatccct gggatcccca 60
   caatgtgtgg agacagaagc caaccaaaga gcgagaaagc atctccaaac cagcagaatc 120
   aggggcagga ggcccgccaa atgcacagca agtcagcctt ggtagtttgc aaatggaaag 180
   atttctttga cccgctggag acttcagctc aggctggatg tcgctaattc acaccatctc 240
   accaaaaggt tctttccctg ccctttggtg aatcgaggca gaagtggctg gtggataact 300
   gggacccaag agttatctca gcgtcgggca ggcatcgaca gctccaggag ccctttccct 360
   gcaggcgggc tggcgggtga gcgattccct cccttcccag gccttcccg 409
<210> 26
   <211> 716
   <212> DNA
   <213> Homo sapiens
<400> 26
   gcgacgggac ttgcccgcgt ctcagcgcct cctgtgtgaa gcgggaccgc tgcggtacct 60
   gccccaggga aggccgcggg gagggttaca gagcccgtag gcactggcgg ggatctttat 120
   acacccccaa aacacacaca tcccacccag tacccagcac ccagcaccca gcacggaggg 180
   cttattatta acaacttcct ggaggccgca ggggcagtgc tggctcactg tgtcccttct 240
   ccctttcagc tctccctaga cctctgtccc aaatcagaaa agaattttac aaactgtgct 300
   ttcctctcgg ggagccccca ccacggcgta acccagcagg caggaatggc acacgtgcag 360
   gaaaatgagt tcaacagcct taccaggtgg tgtcaggatc agcatctgac agtattcttg 420
   ggagaagggg cgccagcctc tcctggcctg ctgtgcgctc tgccaaagtc ttcgctcgac 480
   cccgcacatc ttgctccgtt ccctagtcca gatcccagct ggaggggcca gctgcagcca 540
   cccctaagaa tgaggaaatg ggagcggggt ggggggctgg gttctgtgtt ggcggttgcc 600
   gtggctttgc tccgagttct ggagccaaac cgcccactcc ctgagacggc taggtcacag 660
   aagcagtggg tcagaagcca gaggggacgt ggggagtgcc accctggagc ctaccg 716
<210> 27
   <211> 310
   <212> DNA
   <213> Homo sapiens
<400> 27
   ggagctggcg gagcgcggag tccgcatcgt ctccagaggt aggacgcagc ttttcgccct 60
   gaacccgcgc agcggcacct tggtcaccgc gggtaggata gacagggagg agctctgcga 120
   cagatctcca aactgtgtga caaacctgga gattcttcta gaagatacag tgaagatttt 180
   gcgggtagag gtggaaataa tcgatgttaa tgataaccca cccagttttg ggacagaaca 240
   gagggaaata aaagttgctg aaaatgaaaa tcctggggca agatttcctc ttcctgaagc 300
   ttttgatccg 310
<210> 28
   <211> 372
   <212> DNA
   <213> Homo sapiens
<400> 28
   gcggttctga aaccagatgg taatctggcg ctccgagagg ctggtggctg ccgagatctt 60
   gcgcctcttg tccttggtga tgaacttgtt agccgcatac tcccgctcca gctcccgcaa 120
   ctgccccttg ctgtacggaa tgcgtttctt gcggccgcga cgaaaggcgc aggcgtcagg 180
   agggtgctgc ccgctggagt ctgcgcggcg tgaaagggag ggaggaaaag gcatggtcag 240
   atacccaccc atgcagaccc aggccttgca agccccaagc taagtcatct cacaggtgca 300
   cacaggtcac cctacaggcg cacgtgcaat cctgttcctc caaagcatac caggacagca 360
   ccctggcttc cg 372
<210> 29
   <211> 304
   <212> DNA
   <213> Homo sapiens
<400> 29
   gaagacacac ccaaattctg tccctcttac ttcagggaac atgtccactt tcggcagcat 60
   tacaattttg gcaccaaatg tgctaactgc aattccacca tacaatgcgt aactggaaat 120
   ggaggcaaca tctccgatcc tgaacgatcg atgcgagaat ccaggatatg cacggcttat 180
   tttggccttt tcccactgaa acaagggcca gtattaaaaa tggcacgcta tcctctgttt 240
   cactccctgc ttttaaacgt ctccgatgtt tctccctgag acagggcctc acttccgtca 300
   gccg 304
<210> 30
   <211> 659
   <212> DNA
   <213> Homo sapiens
<400> 30
   gatacactcc tattatatgt gcctatcatc ctgaggagta atttgattca ggtgttctgg 60
   aagtcatgct gtgggctgtg tctgttgaat tcccagcgat gccaggggac acaccctgtg 120
   actccttcct gaattgagtg ctgatatttg attggcttat cgcgcacctg atgagtgggt 180
   ggggtgttcg cggttggtgg gggtgactta cagaagggct gatgcggcca gagagctcgt 240
   catttgaaga ctctctcgga agggatagcg tctttctgca acctgcggtc ccagcagaca 300
   aaccttgtga tcctcgttcc agtcgacatg gaggacgact cactctactt gagaggtgag 360
   tggcagttca accacttttc aaaactcaca tcttctcggc ccgatgcagc ttttgctgaa 420
   atccagcgga cttctctccc tgagaagtca ccactctcat gtgagacccg tgtcgacctc 480
   tgtgatgatt tggctcctgt ggcaagacag cttgctccca gggagaagct tcctctgagt 540
   agcaggagac ctgctgcggt gggggctggg ctccagaata tgggaaatac ctgctacgtg 600
   aacgcttcct tgcagtgcct gacatacaca ccgccccttg ccaactacat gctgtcccg 659
<210> 31
   <211> 462
   <212> DNA
   <213> Homo sapiens
<400> 31
   gcagccgaac gccaggtgcg ctccacgcct gtgcgcgctg atgctggaag cgcgagagga 60
   ggggctggcg gcggcgctca gccgtcagcc aatgagggca gagggcgggg ctatcgggga 120
   tcgcagttcc gacctggcca gccatgccgt tctctgtcga cccctgcagg ccaccaggag 180
   aataacaact tctgctccgt caacatcaac attggcccag gcgactgcga gtggttcgcg 240
   gtgcacgagc actactggga gaccatcagc gctttctgtg atcggtgcgt gccgtcctgc 300
   gcaagtcaga ctgccgcgtc cccgccctcg gtccccagtt cccacctgac ctgtggccac 360
   cccgcaggca cggcgtggac tacttgacgg gttcctggtg gccaatcctg gatgatctct 420
   atgcatccaa tattcctgtg taccgcttcg tgcagcgacc cg 462
<210> 32
   <211> 267
   <212> DNA
   <213> Homo sapiens
<400> 32
   gtgcccacgt ccagggcacg cacaaacgcc atgacttggc ttggcctctc tcttagttat 60
   tcacagctca gcccgatagg cacctctggg gcggcgacgg caaagagggt gcgcttatta 120
   agtgcagctc cacggggact ggcctctctg cacggctgtg tacacctgag cgagacgctc 180
   agtcgctctc taaagccgct tctgcggatg acagacacgg agataaacgt gagaggtggc 240
   ccaccacgac ttgccctcct ttgcccg 267
<210> 33
   <211> 441
   <212> DNA
   <213> Homo sapiens
<400> 33
   gcagcgccgc agctcaggcc tctgcgaatc tgtggtccac acgctgataa acttagaggc 60
   ctctgcactg gggcagaaac cgaaaggcat gacccagacc gagaaagata tttgcatact 120
   gagaaagtgt gaaacacaat tgtcgagact aaaaggagac actgagagga tacgccaatc 180
   aggttagttc agatgtttgt tgagcacact ccatgttcct ggggatccac ccttatagaa 240
   gttacctttt agagagagga gacagacaaa tacgtgtaat gctcaagttg tgataagtac 300
   gatgaagaag tacaaagcag aagggagggc gcggtgtctc acgcctatca tcccagcact 360
   ttgggaggcc gaggcgggcg gatcacttga ggtcaggagt ttgagaccag gtctctacta 420
   aaaatacaaa aattaagtcc g 441
<210> 34
   <211> 243
   <212> DNA
   <213> Homo sapiens
<400> 34
   gcccaggccc aggccgactt gctcaccgtc tacctggtgg tggcgttggc cttggtgtcg 60
   tcgttcttcc tcttctcggt gctcctgttc gtggcggtgc ggctgtgcag gaggagcagg 120
   gaggcctcat tgggtcgctg ctcggtgccc gaggacccct ttccaggcat ctggtggacg 180
   tgagcgacac caggacccta tcccagaggt acaagtatga agtgtttctg acgcgaggct 240
   ccg 243
<210> 35
   <211> 27
   <212> DNA
   <213> Homo sapiens
<400> 35
   gaaggggtta ctaggtgaac actgccg 27
<210> 36
   <211> 85
   <212> DNA
   <213> Homo sapiens
<400> 36
   ggacacagcc tccctggtcc tccccagcgc tccctggttc cccaccgctc ttcgcccgcc     60
   cctcgcccca gcccagtcca acccg                                           85
<210> 37
   <211> 179
   <212> DNA
   <213> Homo sapiens
<400> 37
   gggcggcgac ggcaaagagg gtgcccttat taagtgcagc tccacgggga ctggcctctc     60
   tgcacggctg tgtacacctg agcgagacgc tcagtcgctc tctaaagccg cttctgcgga    120
   tgacagacac ggagataaac gtgagaggtg gcccaccacg acttgccctc ctttgcccg     179
<210> 38
   <211> 287
   <212> DNA
   <213> Homo sapiens
<400> 38
   gaggagctgg ccaagggctc ggtggtgggg aacctcgcta aggatctagg gcttagtgtc     60
   ctggatgtgt cggctcgcga gctgcgagtg agcgcggaga agctgcactt cagcgtagac    120
   gcgcagagcg gggacttact tgtgaaggac cgaatagacc gtgagcaaat atgcaaagag    180
   agaagaagat gtgagttgca attggaagct gtggtggaaa atcctttaaa tatttttcat    240
   gtcattgtgg tgattgagga tgttaatgac cacgcccctc aattccg                 287
<210> 39
   <211> 520
   <212> DNA
   <213> Homo sapiens
<400> 39
   gcgggccttc ctttcctctt atctgatcct gggctcccag ctggagaggc ggaggcagct 60
   ccagggggct ggaagtggaa gcgcagcggc agaaggagag ggagagagaa agagagagag 120
   gctaattaaa aaaggatact ccgagggaag agagcaaggg cggtgcgccg ccaaggacca 180
   actagcggcg gagcttcgat cttgcctagg cgcggagagc tcccaacctg ggctggaacc 240
   ttgcccagca caggtcagtt cgtctttctc tgctcttctt tggctcggct tcgaagtcca 300
   ttcatgagca aggaaaagtg gaggcagcga gccacctgca gacgcaatgc agttccatgg 360
   actttctttt cacgcgcgcg cccagaatgg actggggacc tttgggacgg gatgggacgg 420
   gtacacctgg atgccttttc gcggggttca cggtgtaggg agattagagg cattgggtga 480
   tgagagcgca gggatagtgg aaataccatg tgctgctccg 520
<210> 40
   <211> 165
   <212> DNA
   <213> Homo sapiens
<400> 40
   gcagtccagg gaagggctgg ctcagggtga ctctccccag ttcagtcctc cgtttgctgg 60
   agacctgggt gagcctcagt ccctttcaaa ctgtttcgct ccgcgtgcag atttttggag 120
   caattctttc ctcctgacgc gataacagac cgcgaagcat ccccg 165
<210> 41
   <211> 281
   <212> DNA
   <213> Homo sapiens
<400> 41
   gctggctttg acacttgctt ccaaaggcga cctgcaccca gggccaggac cacggcatct 60
   gagcctcctc ccccagccgc caaccccatt cccaatggag gagcccagga gaactggggg 120
   cggccaaggc agcccttccc tgctccgcag atccagagga tgcccacagc ccccttcccc     180
   tcccgccttt cccacactcc ctgtagcaat tcacacaagc ggggagggga cgaaactgct     240
   gaatccagca gatagcttgt ggcggggtaa tcctcggtcc g 281
<210> 42
   <211> 710
   <212> DNA
   <213> Homo sapiens
<400> 42
   gtgctggggc tcctgggtgg ggctgtcact gcctgagttt gggggcctgc cctccttgtc      60
   ccccccgaat ctccccctcc ctaatccctt gggggggatc aggagagtag gggttcctag     120
   tttcatttct cctgcatgaa tcagatcatc ttctcacccc acgggagccc tgggatgccg     180
   aggctgggtg ggggcggtag aataatcctc cgtggcacga ggaagcgctg gaataatcct     240
   ccatggcacg agggagcggt ggaataatcc tccgtggcac gggggtgggt ggaataatcc     300
   tccgtggcat ggggggttgg tgggataatc ctccatggca aaagggagcg gtggaataat     360
   cctccgtggc acggaggggg tggaatgatc ctccatggca cgagggggca gtggaataat      420
   cctccgtggc acgagggggc agtggaataa tcctccgtgg cacgaggacg gtcactgcat     480
   tcgtcactct tagctgcagt gcgtgtgaca gtcgccaggg ctgctgtttg aggaacgggg     540
   gttgcctgcg tctgctgtgc cccaggtggt tcgtagggtg ctttttactc cttgccaaag     600
   cccacacagc cccgcaaggc aaacagtgtt caactctttt catggctgaa aaattgggac     660
   atcagagagg ttaagacact ggccaggacc acctggcagg gaagcttccg                710
<210> 43
   <211> 340
   <212> DNA
   <213> Homo sapiens
<400> 43
   gctgtgcccc agggttctaa aggcggtggt ctcagagcag cggctgaccc gtgacaccgc      60
   gtgtgcaccg cagtgcgcca ggtacggctc gtacggggcc tgcaggcagt ggaggcacgg     120
   gagcagggca cggctaccat ggaggtgcag ctgtcgcatg cggacgtgga tggcagctgg     180
   actcgtgacg gtctgcggtt ccagcagggg cccacgtgcc acctggctgt gcggggcccc     240
   atgcacaccc tcacactctc ggggctgcgg ccagaggata gtggccttat ggtcttcaag     300
   gccgaaggag tgcacacgtc ggcgcggctc gtggtcaccg                           340
<210> 44
   <211> 291
   <212> DNA
   <213> Homo sapiens
<400> 44
   gctcaggtga cctcaccagg gcctcacaga tcgaagtctc ctgccccagg acctgcagca 60
   ctgtagcgta gcaactacat tgccctgtgt ctgaactcat ttgaggcggg ctagggcttt 120
   tgaagtgtta cttctttccc tcccctcaca gcggatcgtg tgaagaaggg atttgacttc 180
   cagtggcccc aacccgataa accgatgttc ttctacgtga cccagggcca agaggagatt 240
   gccagctcgg gcacctccta cctgaacagg tgagcaggga caggcccacc g 291
<210> 45
   <211> 491
   <212> DNA
   <213> Homo sapiens
<400> 45
   gaggagctgg ccaagggctc ggtggtgggg aacctcgcta aggatctagg gctcagtgtc 60
   ctggatgtgt cggctcgcaa gctgcgagtg agcgcggaga agctgcactt cagcgtagac 120
   gcggagagcg gggacttact tgtgaagaac cgaatagacc gtgagcaaat atgcaaagag 180
   agaagaagat gtgagttgca attggaagct gtggtggaaa atcctttaaa tatttttcat 240
   gtcattgtgg tgattgagga tgttaatgac cacgcccctc aatttgataa aaaggaaata 300
   catttagaaa ttttcgaatc tgcatccgct ggtacacgac tatcgcttga ccctgccacg 360
   gatcctgata taaacataaa ctcaattaaa gattataaga taaactctaa tccttatttt 420
   tcattaatgg ttagagttaa ttccgatggt ggcaaatacc cagagttatc tctggagaaa 480
   ctcctagacc g 491
<210> 46
   <211> 287
   <212> DNA
   <213> Homo sapiens
<400> 46
   gagaaaagaa aaagaactca tttcccatga gctcttagag ccactgaaag ctcagaacaa 60
   aaccaactga gcagggtttc aagtattctt tttggtttca ttttctcctc aaattttctg 120
   aagaacagct gagagtttag caaatacggg tgcaaacgat tctgcctgcc acgtcaaccc 180
   tgcctcgggt gtcatctgac gaggatgcca agctacaagg gctgcggtga gactcagaga 240
   cacgctacca atggcaccgc ggccccacct cgccgaagca gctgccg 287
<210> 47
   <211> 495
   <212> DNA
   <213> Homo sapiens
<400> 47
   gcgctctgtg agcagatccg ctacaggatt cccgaggaaa tgcccaaggg ctccgtagtg     60
   gggaacctcg ccacggacct ggggttcagc gtccaggagt taccgactcg aaaactgcgc    120
   gtcagttcgg agaagcctta cttcaccgtg agcgcagaga gcggggagtt gcttgtgagc    180
   agcaggctag acagggagga gatatgcggg aagaagccag cttgtgctct ggaatttgag    240
   gctgttgctg aaaatccact gaacttttat cacgtgaatg tggagatcga ggacattaat    300
   gaccacacgc caaaattcac gcaaaattcc tttgagctgc aaataagtga gtctgcacag    360
   cctggcacaa gatttatact agaagtagca gaagatgcag atattggctt aaactctctg    420
   cagaagtata aactctctct taacccaagt ttctcattaa taattaagga gaaacaggat    480
   ggtagtaaat acccg                                                     495
<210> 48
   <211> 1492
   <212> DNA
   <213> Homo sapiens
<400> 48
   gatcaagatt agaggctctg ctctccgtgt tcacagcgga ccttgattta atgtcataca      60
   attaaggcac gcggtgaatg ccaagagcgg agcctacggc tgcacttgaa ggacaccaaa     120
   gcatctcagg gtcagaaagg ggaaaaagca attgcaggga atttaggggg tagtaaaagg     180
   aacccatctc ttgccgcata aatgcccccc acccccaccc aggactgatt ctggaagcaa     240
   cctagtgttc gaaagggaaa ggctcctact tttccattac agccgcggaa atccgcaggc     300
   aaatctccga ggagaatttt agggaagctt cattgacagc tgtctggaga gcagtagttc     360
   ccgcctgtgc aaatattcca gagagttaaa tcatttagaa agcactagtt ctttcaagaa     420
   caggtagtgt gattgtctgc agtcctgggc caagctggga aaacaaaaca gcagagagag     480
   gtgtaacatt aaatagcgtc ctgaccacac tgcgcaagaa acagcaaatg aaacgtccaa     540
   actttggaaa gatctgaaga acacttgccg cccgcatttc cccttgactt ctcccttttt     600
   cttcccaatc tcgaggcgtt catgcctggc tttaagacgc caagataact ctcttgtttt     660
   ggcattgtcg caatgtccaa atcggtcaaa ggagtgggat aaggtgaaag gaccaggtca     720
   gaaactttag aaggactcgg taaattaatt tgtttggaag ccagtgaggt ggactatgtt     780
   gaggtagcag ttccgcacca cgactttgga gccaaagaaa aacccaggcc gaaaaagcga     840
   gccttgatgt ctttgttctg ccttggcctg caggggttgg gaacaaagga gaggagctaa     900
   gaggatgcac caaagtagcc gcgccctccc tgcctggcgc tgtccgcggt gctgaccgtg     960
   gcccttttct ctctccgtaa tcaaactgga aaagcaatag gaccgtcccg tccctactca    1020
   aaagaagagt aggatattag gtaatttgaa aaataacatt taaaaagaga aaaaggtcac    1080
   atacagcaaa acaatatttt actgaaatcg ttccctcaag ctggttgcta cccttgcctg    1140
   acactttttg gtcttcacgt aggaatgcac acatacatac attgtaagtg tatggggaaa    1200
   ccatttccac tttatttagg ctgaaaccta aatcgcagga ttgcaagaag gaaattgagg    1260
   agaatgaagg ctttattatt atccttttct ttcttaaatc atcttcctta aaacagaaac    1320
   ataaggtcct cagccgcgga ttttagtgct tacttggtca ccaaagggac gtgcaatgga    1380
   ctcaagtgtg ggcctcgagg cttaactagg aaggggtggg tcggggaccc accaggtgaa    1440
   acagggcagg agcccatgca gcttgccacc ttcggccagg ccttgcagcc cg            1492
<210> 49
   <211> 365
   <212> DNA
   <213> Homo sapiens
<400> 49
   gagggagctc cagacccctc ccctcgcgcc cgcggccgct gcgcggaggt ctgcggaggg 60
   cgcctggctc ggtcggtcgc tccttccttg gcgggccctc gccgacccac ggtgctcagc 120
   cagccccatt cttggcattc accgcgtgcc ttaattgtat ggacatttaa atcaaggtcc 180
   gctgtgaaca cggagagaga ggcctttctc ctgaggaagg aaaggaggaa ggaaggaagg 240
   aaaggtgaaa gaaaggaaga ggggtgggta gaagatggaa taagaaaacc aggaaaaaga 300
   aataaaaagc ggcgcgtgtg cgtgcgcact gacagcgggg agagggatgg gggtggggaa 360
   cgccg 365
<210> 50
   <211> 303
   <212> DNA
   <213> Homo sapiens
<400> 50
   gtcgcccgtc agcgggagta ggagggaagg gacacgagtg gagttgaggg ggagggtgaa 60
   gagagaaatg aagtccgaga caaaacaaca acaaaaacct cagacacgga gatacagaca 120
   cgacagagac cgaaaaaggc gtggaaagga cgcgatgacc cgtggcgtcg aagtcgggga 180
   gttgaccccg atccagaccc aaaaagtttc tggtgcccca tttcccgctc tcccattcgg 240
   gccaggagca ggagttccgc tggtcccagg tggaagggac gcgcgggctt ttcgtgccac 300
   ccg 303
<210> 51
   <211> 275
   <212> DNA
   <213> Homo sapiens
<400> 51
   gggcgccctg gaccacatag tcaggatgac cacgagcctc taacagcccc gccgtggcct 60
   tcgcgtgcca gaccgtgtgc cccgagagga ccctgtgcgt ggggaagagg cggctggcgg 120
   tgcaggaaat cctggcgact caggaaattc tggcggcgcg gcgtggggtc ggtgggggcg 180
   gcaggcgcag gtggcgggcg aaacggaggg cgcagagcag cacaagcggc ctggcctaga 240
   ggcggcgggc tcccgtgagg aatccccaag agccg 275
<210> 52
   <211> 374
   <212> DNA
   <213> Homo sapiens
<400> 52
   ggaggcagag gttgcagtga gccgagatcg cgccactgca ctctagcctg ggccacagag 60
   caagactcca tctcgggaaa aaaaaaaaaa agtaattatt ggatggagaa aagggcagac 120
   aaacggggca gagaagtgca taggcagata gaaacaagaa caagatagac agagcacatg 180
   gatgaacaga cacacggaca gacacgtggg acacaccgat gggcagatag agtgcacaga 240
   gaaacagacg gggcaagtgg atgggcaggt ggaaagagag gcagggccca cggacaaaca 300
   gactgggatg gatgcataga cagacggatc gatcgggtgg atgggctcac ttgcaagtgc 360
   gctcgcggcc accg 374
<210> 53
   <211> 275
   <212> DNA
   <213> Homo sapiens
<400> 53
   gctcttgggg attcctcacg ggagcccgcc gcctctaggc caggccgctt gtgctgctct 60
   gcgccctccg tttcgcccgc cacctgcgcc tgccgccccc accgacccca cgccacgccg 120
   ccagaatttc ctgagtcgcc aggatttcct gcaccgccag ccgcctcttc cccacgcaca 180
   gggtgctctc ggggcacacg gtctggcacg cgaaggccac ggcggtgctg ttagaggctg 240
   gtggtcatcc tgactatgtg gtccagggcg ccccg 275
<210> 54
   <211> 411
   <212> DNA
   <213> Homo sapiens
<400> 54
   gctcaaggag cgctttgagg aggaggcgcg gttgcgcgac gacactgagg cggccatccg 60
   cgcgctgcgc aaagacatcg aggaggcgtc gctggtcaag gtggagctgg acaagaaggt 120
   gcagtcgctg caggatgagg tggccttcct gcggagcaac cacgaggagg aggtggccga 180
   ccttctggcc cagatccagg catcgcacat cacggtggag cgcaaagact acctgaagac 240
   agacatctcg acggcgctga aggaaatccg ctcccagctc gaaagccact cagaccagaa 300
   tatgcaccag gccgaagagt ggttcaaatg ccgctacgcc aagctcaccg aggcggccga 360
   gcagaacaag gaggccatcc gctccgccaa ggaagagatc gccgagtacc g 411
<210> 55
   <211> 387
   <212> DNA
   <213> Homo sapiens
<400> 55
   gagcgcgcct ggcgctgtcc gtggtgctga gaggtgggga ggccagcgtg gcggcggcgc 60
   tgggcggaat gtgggcgaga catctccgca aggcttggcg gtccttcccc acggccctca 120
   tcctctcatt gccaccaggc ctcctcccac agctgttcct gaatccgctg cattggcctc 180
   taaaaccaaa aatcagggag agggggcgac agactaggag tttgcgctct cttccccatg 240
   cgctctgcgc aggagttctc gagtgtgcgc agtctggcac gtgagtcagc gcggggtcta 300
   cgcggattgg tgcgggggaa ggggagaagg atggtcccat tccccctcca tcctgctttc 360
   cctcagcggt ccccttattg gaaaccg 387
<210> 56
   <211> 227
   <212> DNA
   <213> Homo sapiens
<400> 56
   gagcgcgcgg ggacttctct tgcttcctga agcgcctctg gctcggtggc cgtggtgcgc 60
   ccctccagtc tacgcgatgg gcacaggaag cggctggtgc ccgctgacag ccagggctgt 120
   tctcgggagt ctgcggcgcg ccagatggca gcgacagcgg ctgtgtctgg ctcggagcga 180
   gagaagagag caagccgcca ctgaggggct ggggcaggca gtcgccg 227
<210> 57
   <211> 521
   <212> DNA
   <213> Homo sapiens
<400> 57
   ggcaggtgat gggcagtcat caagaaggac ttctgggtac ctggtttaga caatgggtgg 60
   gcagtggaga cttctcaaga caggggatga agcagtcatc aggtttgggg taaaagtcag 120
   gacaaactgg cttcacgttg gttggttttg aagaacctgt gagtcttcca agtagataga 180
   gccaaacaca ggcctggaac tctgcagaga aggcaggagc tagaaatagg cgtggagccc 240
   ttgtgagggt gcagcaccac cagggactgt gcgcaggtgc aaagaggcca agaatggaac 300
   tccagggagc tgagagagag agagagggaa agaaagaaag agagagagag agaagaggtc 360
   ggggaagaca gggccgaact ggatgacagt gatcatgggg aggccacaag gaccaactgc 420
   cccacgaggc gtgtggacag tgatacctaa tactacagaa accgtcctag gatgcaacac 480
   ccagtggtgt ggggactggg tgacttttgc actcctttcc g 521
<210> 58
   <211> 545
   <212> DNA
   <213> Homo sapiens
<400> 58
   gaggtcgcgc cgccttgggc ttgaattcag tccttcctta ctactcgtgg ctttgcttac 60
   atcatttaac tctccatttc cttgtctata cagtgggctc ataaggaggc ggtaaggaga 120
   tgatgatgtc tttgaagagt ttaggacagg acctggtgtc tcccgtacgc ttggtggggg 180
   tggcggaggt gatgcgttgt taacaggaga aaccgacctt ccagggaggc ccagggaggc 240
   agcggagagc gaagtcctgc tcggcggaga gacctcgagg agagtatggg gaaaggaatg 300
   aatgctgcgg agcgcccctc tgggctccac ccaagcctcg gaggcgggac ggtgggctcc 360
   gtcccgaccc cttaggcagc tggaccgata cctcctggat cagaccccac aggaagactc 420
   gcgtggggcc cgatatgtgt acttcaaact ctgagcggcc accctcagcc aactggccag 480
   tggatgcgaa tcgtgggccc tgaggggcga gggcgctcgg aactgcatgc ctgtgcacgg 540
   tgccg 545
<210> 59
   <211> 247
   <212> DNA
   <213> Homo sapiens
<400> 59
   gaacctcgct ggcgatctgg gttttttgcg cgtgggagta aacatttccc aagtccctca 60
   cactctgggc tgctagtgag gttctgccag aattccttag gccgccgccc actccccttt 120
   ctctgaacgt cactaatttc ctggaaataa ttccaggcac gcatcgctcc attaaagtta 180
   atgaagcacg tgtccgtgac agcagccagc gccagcgccc gcgggagagc cccgcgcgcg 240
   cttcccg 247
<210> 60
   <211> 247
   <212> DNA
   <213> Homo sapiens
<400> 60
   ggaagcgcgc gcggggctct cccgcgggcg ctggcgctgg ctgctgtcac ggacacgtgc 60
   ttcattaact ttaatggagc gatgcgtgcc tggaattatt tccaggaaat tagtgacgtt 120
   cagagaaagg ggagtgggcg gcggcctaag gaattctggc agaacctcac tagcagccca 180
   gagtgtgagg gacttgggaa acgtttactc ccacgcgcaa aaaacccaga tcgccagcga 240
   ggttccg 247
<210> 61
   <211> 506
   <212> DNA
   <213> Homo sapiens
<400> 61
   gtggataata agcattgcct cacaggtaac cactgttact aggtggataa taagtactgc     60
   ctcatgggta accactgtta cccgatggat aataagtact gcctcgtgga taaccacggt    120
   taccctgtgg aaaataagtg ttgcctcgtg ggtgggtaag cactgttacc tggtggataa    180
   taagtgttgc ctcttgtgta accactgtta cctgctgcat aataagtatt gcctcgtggg    240
   taaccactat tacccagtgg ataataagta ttgcttcgtg ggtaatcact gttacctgct    300
   gcacaataag tgttgcctat tgggtaacca ctgttacctg gtggataata agtattgcct    360
   catgggtaac cactgttaca cactggataa caagtgttgc ctttggtaac cacagttacc    420
   cagtggatga taagtgttgc ctcgtgggga gccactgtta cccagcaggt aataagtgtt    480
   gtctcatggg gaaccactgt tacccg                                         506
<210> 62
   <211> 352
   <212> DNA
   <213> Homo sapiens
<400> 62
   ggggcctcag cccctcaggc agcagggtga gaaactccca aaagctcacc caaaccacgc     60
   tcccatccct gggggtgcag ttcttcctcc tcccacctac gcacatgtct cccatttccc    120
   ctaactggaa aggctcatgc ttgacaatca cagggaagca ggcagcgctc gctaaaaccc    180
   agatgcctgt gaacagacag cgccagccat tcacacccca gtggatgctg gcttattaga    240
   tttgattggc agcctctgga gtaggcaggg tgggctatac agggcgtcta ggaagacaga    300
   taggtcacgg cggagacagg gctggccccc tcgctgcatc tccgagggtc cg            352
<210> 63
   <211> 291
   <212> DNA
   <213> Homo sapiens
<400> 63
   ggctgctacc ctcaacatgt gtgcacctcc ccgccaactg cctctgagct ggcagtcagg     60
   cagcagcact agctactcta ttgccattga ctctgttaat taaaactgac acacacgtgc 120
   aaacaacaac caccccatcc ccagcaaatt cgaaaaacaa agccaaacaa aacacttcaa 180
   actattttct cctggacagc ctttgacctc cttgtaggca aagctgggag aaatacctct 240
   ccttaatggc ttaaatcgat caaaacacct cctcgattga taaacatgcc g 291
<210> 64
   <211> 259
   <212> DNA
   <213> Homo sapiens
<400> 64
   gtttggggga cgccaattcg cctaagaaaa ccctggcaga agagcgcgga cccttcacta 60
   caaacctcac gtcagggtta cagccacatt taggaacctc ttcggaaaag ctgagaaatc 120
   actgttttgc aaaaagcctt ctgtactgtg atggggcttt gtggtgagag gaacctctga 180
   gaagcctcgt gcggcttgag tttagagtca cgccctgccc agcgacattc tcccgcgcac 240
   gggagaacct gcacttccg 259
<210> 65
   <211> 4870
   <212> DNA
   <213> Homo sapiens
<400> 65
   gggaccttgc ctgtacaaca ctcacagtct aatttgtggt ggaagtggat atggaaaagt 60
   aagcaacagt cccacatggt ctggcactta ctaggttatg tgccatgtgc tgtgaaagca 120
   cagagaagca gcactgaaac ccaagaggaa gacaggagga agatattcca gaaggagacc 180
   tcatatttat acattacctc aaaagccaag ggtgtctgcc cttcctctga ttaaactgat 240
   caccagaaat atggtcaagc caagaaaaag ccgtttaaga aataaggcga gtttatatgg 300
   aaggaaatgt agaaggaaaa aaaacatttt cagtgttgct ccctgttttc tttatcgatc 360
   cagatacaag tcgttgatga aataaagtag gaggaaaaaa gatgtaaaaa ggaggagaca 420
   ataatataaa cactatcatc accaaaacta aaagtaacat gttcctatga gcagtgccct 480
   gactaggaca gaacctggcc aagagtggat tttccagata tgtttattct ggtaatgaga 540
   gtgcacattg agttaaactt tcaagaagga tttttgcaac caaattcaag aatcttgtgc 600
   aattcattca gtctctgtct aggaatgtat ccaaagtatg gggaaaactc aaagagttgc 660
   tgagtaagtg tgttaaacaa gtattattta taatttggaa aaatggaaat aacctaatgt 720
   atagtagtag aaaattgtta attacgttta atacatccat aggatggaat tttgtgcaat 780
   cttgaaaatt gagttggagg caatagatca gaacgaacac attcttgctg tgggctgtgt 840
   ctttcttggg gtgtacactt tttcagccag agctaaagct ccctgttttc acatcattac 900
   tttctctccc cccacttttt tcctaaccac aacccttcat atacatttgt aggcttttct    960
   ctccatttat ttatcttagt aacaaaccct aattcattcc tcttctaatt aattttcata   1020
   ctggatatat ttcaaagtag gttttcatga ctttggaaac aaattaaaaa actgtgtgta   1080
   tgtatgtgca ggcacacaca acttgtacat gtagtgacta tgtaaaaata tacagctaac   1140
   aggcaacgat gataacccct agaaaagaag aggactgcat gacaagaaaa ctgaagtaaa   1200
   aggaggcttc ctttcactga ttattctttg gtactagttt taatatcata aacattgtct   1260
   tagtctgttc agcccactgt aacaaaacta cacaccaggt ggcttttaaa cagcaggaat   1320
   ttatttctca tggttccaga ggctgagaag tccaagatta aggcagcagc agattccgtg   1380
   tctggtgaga gcccgctttc tgcctcatag acagagcctt cttgttttgt tcttacatgg   1440
   tagaagaggt gggggaggtc tctctcagac tttgtgtgtg tgtgtgtgtg tgtgtgtgtg   1500
   tgtgtgtgtg tgtgcgcgcg cgcgtgcgtg cgtgctggga ttacaggcgt gagccaccgc   1560
   acccagcctc aggactcttt tataaggaca ctaacctcat tcatgagggc cccaccctca   1620
   ttacctaatc acctcccaaa gaccccacct cctttcaaca tccgtcattt caaaatctga   1680
   ttctgagggg acacaagtgt tcagcctata gcaaatgtat tatctggtca atatataaat   1740
   aaatatatct tgtaaatgga atgtagaaat aatctgatag aacatgatca ttttcatata   1800
   agcaagcata gacccagaaa aatgaagcaa ttgtcccaag gtcacataat aaattagtgc   1860
   aagtgctgat attaaaagga aaaggatctg gcagggacag aaacaaattt accgagaatc   1920
   tggcatctaa ttcacttaat acctctgaga aaaacgtttt taaagatgaa gaaattgatg   1980
   ttcagagaca tcatgtaatg ctaaaggcaa cacagctgag gagaagctca ggttcaaatt   2040
   tgattctacc atccaactct ctgggctctg ctctttttga gtctttatgc tgctctgccc   2100
   ctctcaatcc catagtcagc taaattctgt gttgtttatc tagtccatgc tgtccaagac   2160
   acaatccctc attcttttga attcaccctt attaatctgc accatcccaa agggcaacac   2220
   tctacatcca tccaaacttt actttttcta aatagaagag aatcttccta aagagagact   2280
   cgtaagtcaa cacattttca cagcatttca catatcattg tttcacaaag aaatctacta   2340
   tttataatct ttttaaactg aaaagctact ctaccttcct ttatacagta ccattaggct    2400
   gtgggcttat aaacctgagc catatcattt ctcccaccct ttattggaag gtgcagcagt    2460
   cagggtttct gcaaaaatca gatgatccac ataaatacag taattgaaaa tacaataaag   2520
   aaactatctg catttgttta agcaaaagtg gttgagagaa accacaaaga gatggtcaag   2580
   ctctctgggt tagcaacagc agggagacat tatcactcct tggcatgaag agacgaggga   2640
   gggggcagtt attataaccc agtaagaaat gaggcattca gttactgcca ctgttgccca   2700
   gctaggagga agtcagggaa ataaatagca taatatcaca ctcctcctac cctctgattt   2760
   cctctgctga tgttgccatt ggtctaaccc aactgaaacc agagggcaag gggaccagtt   2820
   aaggaatacc tcccaggatt cggcgtgagg cgccaagggg cagaaagttg atctgtgaaa   2880
   ccatgcaaaa agtaccccac atggtttctg ctgcatgcag cattatacca ttcagtggtc   2940
   attccctcca ctgactgact gctgtgtgga tctcacctga ctactgactt gtaacacaag   3000
   gctctctgtg gttctgaaat attctccaga tccataactg accaccctag gcggtggact   3060
   tctccatctt gaagtttttt tgttgttgtt gttgtttaga tggagtcttg ctccattgcc   3120
   caggctggag tgcagtggca tgatcttggc tcgctgcaac ctccacctcc tgggttcaag   3180
   cgattttcct ccctcagtct cctgagtagc tgagagtaca tgcatgagcc actgcatttg   3240
   gtccccatct tgacttttga aaacagggca atgattgtat gtgattgcat gtgaaataat   3300
   tcccaaattc tgttcttcat aaccttgaca ttttctataa gttcttccac ccagcatcac   3360
   taaaaccctt caagtccaag tccctgaagc tccaccccac tgtcttaaga ggtaccatac   3420
   atgctgctcc ctgggtccta acacacttcc cctgcacctt cttcaaaatg cttcaagaat   3480
   ttccctggtc atttggccaa ggccatctca acaaaaaatt ctatatactt cctacctact   3540
   tgcaaaaatg cgacatttaa aattagcctt tgaaaagttg catgtaattt ctgaggacag   3600
   aatcagattt taacatccta ggccagctca catttgaaat gaaatcttcc agaggctgag   3660
   ggaggaagaa agtgagccat aatgaaaagc agccagtact ggaaataaag agcctggatt   3720
   ttagtcgcag ttccactgct gagcagctga gcgacctcgg ggaaggtaca taagcttgag   3780
   tctatcctca gaaatagcgg ggaactgagt gaaatgatcc tcaagactta ctgtgtgagt   3840
   attcaaagct cttaagaatg tttgtaaatt aaagaaccct taaaatgggg atgatgaacc   3900
   tgtttgagga aaaattatat ttcttccatg taaccagtgg agtccaaatc acttatggga   3960
   gaaacagtgt ttctttcctg agcattgccc ttttctcagg agaatgcatt tttgttcaag   4020
   attttcttta gagcaacttt gacgtcctta ttcctcaggc tataaattaa tgggttgagc   4080
   atgggcacca cagtggtata gaatagggaa gacaccttgc cctggttcat agctaaaaga   4140
   gaaaagggtt tgaggtacat gaatgctcct gacccaaaga acagagaaac tgcaattatg   4200
   tgggagctgc aggtgctgaa ggctttggac ctgccctccg tggaatcaat gtggaagatg   4260
   ctggagagaa tgagagcata ggaaatgaag atggtgactg tgggcacacc aatatcaatg   4320
   cccacaacaa caaacactac aagctcattc acataggtgc tggtgcaagc acactcaaga   4380
   aggggaagga tgtcacacat gtagtggttg acaaggttat tggcacagaa ggtcacaccc   4440
   atcatgcacg ctgtgtgggc catggcccca gcaaacccca tcccatagac acccaacaaa   4500
   aggagaaaac acacctgggg agacatggtg accatgtaca acagtgggtt acagatggcc   4560
   acatagcggt catacgccat tgctgacagg atgaaggact cagagacaac aaagaaaaga 4620
   aagaagaaga gctgagtcat acaccctgcg taggagatgc tgttcttctt taagacaaag 4680
   ctcatcagca ttttgggagt gataacactg gaatagcaga aatctatgaa ggacaagtta 4740
   tagaggaaga agtacatagg ggtgtgcaag tgagagttga gccttatcag ggttatcaag 4800
   cccaggttcc ccaccacagt gaccacgtag aagcctagaa acaggaagaa gagggggatc 4860
   tggactcccg 4870
<210> 66
   <211> 355
   <212> DNA
   <213> Homo sapiens
<400> 66
   gcacgcgcga gcgtgctcct gcccgtggaa ggggggaaat cggcgactgg gttgcaggga 60
   gaatgcgatg ataaatggcc ctgcgtctgc tttctgactt tctctcctgg acccagctcg 120
   taaccccaga ggctccccaa cctgcctgcc gccaagcacg gctgtgcgca aaaggccacc 180
   agccgcttcc tgcagccaga gctaaggttc actcgcctct tagaaaggcg cggcccctcc 240
   cctgctgctg cttgatgacg atccccatcg tctcctcggc ctgcctgtgt ccgagaaaga 300
   gttgagactg gactcgaacc cctgcgcgcc aaggggaggg cgtcaccatg ctccg 355
<210> 67
   <211> 306
   <212> DNA
   <213> Homo sapiens
<400> 67
   gagccgagga cccagcgcca agccaggagc aaagcagcgc gccaggctgg agctgttccg 60
   tcattaccgc cacgtctgcg cttcggttgt gagaaccgat tcgttaccct aacctcttag 120
   gactaagagc cagtcttctg gtggcgctcg ctttcactta ctttttattt ccctttctat 180
   tcccgcgagc tagggggaga aagtgtaaat cccgcccgct tctccagggc tgaaaatgga 240
   gaggctgagg cggcttggag agtgcgcact gcgcatgctc cgcagagcat ccccgcaggt 300
   caaccg 306
<210> 68
   <211> 349
   <212> DNA
   <213> Homo sapiens
<400> 68
   gaacgtaatg tgtcaataaa aaatatacag gtttcacaaa ggcaaaatgc caaaatctca 60
   catgaaacac tcgtttgcat aaaaaaggaa gcagctgccc ccttaaacca actttttccc 120
   aatgctcacc cgaacacggt ggagtcgcca ggtccgtggg gctacaggat cttggggacg 180
   gagtcaatcg aacgcgaacg gcaaaattct tttccctctg aaacacaatt cacaaacgtt 240
   gccttggaga cttatttttt aaggcctcag aacgcttgga cacttttgaa gacgggaaca 300
   cacggctgaa aacagccctc ggacgcgccc atactgcact tcccacccg 349
<210> 69
   <211> 339
   <212> DNA
   <213> Homo sapiens
<400> 69
   gccactggga agcgggcctc ccacgggctc ggagctggaa gggtcgtggg gaggcccgcc 60
   cttcacgtcc atcttcgttt ggcccctctg agttagggga aatacatcac agtgcttggg 120
   ccatagaacc aggcagctcc gagctgaccg atcttcccga aagcccgtgc ccaggggact 180
   ccagtgggag ggtctcattc tttaaggtct ctggcgtgct gttccagagt ttacccatca 240
   acaattctta ctttaatttt ttggagccag ggtctcgctc tgtcgcccag gctggagtgc 300
   agtggtgcga tcatggctca ctgcaacctc gacctcccg 339
<210> 70
   <211> 456
   <212> DNA
   <213> Homo sapiens
<400> 70
   gcctctcgca ggacctgggg gccctgaaga tccccgagca gtaccgcatg accatctggc 60
   ggggcctgca ggacctgaag cagggccacg actacagcac cgcgcagcag ctgctccgct 120
   ctagcaacgc ggccaccatc tccatcggcg gctcagggga actgcagcgc cagcgggtca 180
   tggaggccgt gcacttccgc gtgcgccaca ccatcaccat ccccaaccgc ggcggcccag 240
   gcggcggccc tgacgagtgg gcggacttcg gcttcgacct gcccgactgc aaggcccgca 300
   agcagcccat caaggaggag ttcacggagg ccgagatcca ctgagggcct cgcctggctg 360
   cagcctgcgc caccgcccag agacccaagc tgcctcccct ctccttcctg tgtgtccaaa 420
   actgcctcag gaggcaggac cttcgggctg tgcccg 456
<210> 71
   <211> 483
   <212> DNA
   <213> Homo sapiens
<400> 71
   ggctgtctgg ctgctgttca acagggagtc cagctgcctg gggtcctaca gaacggggct      60
   gggcaggcat tcttctctgt ccaggctctg gcttcagggc caggaaggca ggagttgttg     120
   aggtgcctta gttttaacag tggtcttggt catgtgtgga ggctcaggat tagagtcctc     180
   accctgtctg ctgtacccga cttggttggt cccacactga actcatgact tgccatagag     240
   cgagggcctc cctgacggga gctggggagc aggccctggg acagctgagc ctaactcagg     300
   cttcgtcttt ctgctccacc cttccccaaa gctgatcctt tcactgtgca ggcctgctga     360
   gacctccttg cttctcggtg cccccccttc tctaaggaca gacaccctga gcctgttgtg     420
   catggcttct gccacttctc agcaagacct cctaatcaaa tcctccaagg ttgtccgtgt     480
   ccg                                                                   483
<210> 72
   <211> 347
   <212> DNA
   <213> Homo sapiens
<400> 72
   gagcggggtg gaaggagtgt caaggtgact gtgagatgac agacacgtag gggagcccca      60
   cagaggaccc ccaagacacc ctctctgttg ccatcagcct gagtcatcct ctatctgaaa     120
   aacttccagg actgaagttc gagttggaag cacgcgttct ccttggtgaa gtccgtcctg     180
   tcccaggagg gctgcgctag gtctctcctc tggctccccc tcctcgggtc ctcgtggctc     240
   tctgtcgtgt gaagcggggg gtcaggagcg cacaaatgtt tatactccac gtgagtcact     300
   tgcacagatg acgggtgatg ttttcctcgc caaggctgat ggcaccg                   347
<210> 73
   <211> 371
   <212> DNA
   <213> Homo sapiens
<400> 73
   gtcatagcgc tgcgcagcgg gaccgttctg ccccgctgcc ctcgaacagc ccgtccgcgc     60
   tccattgccc ctccaggtgc cgttattttg ccttttaacc tggggcggga ggttttagac    120
   gaggctgcaa gtctgggcca gcggggacct gagggtccct tgcaaccagc acccgcgacg    180
   gtgtctacac tggaattcca aggaaaagag ggctccaggg gcgcgaccaa ggtttggtct    240
   tctgctgagc gctgctggat gaatcgccag ccttcgcccg cggccccctc ctcctcatcc    300
   ctgaccccaa ctcgacaaga tcccccgagc gcaggcccaa actccgcaca gcttgtgggc    360
   cgtgaggtcc g                                                         371
<210> 74
   <211> 466
   <212> DNA
   <213> Homo sapiens
<400> 74
   gggtctgtcg gggtcgggtc tgtttctccg cagcggaggt ctgtgtcctg gcttgggggt     60
   tgcaggtgaa ttccagcctc ctcgtgagga ggggagcaca gacctgtgta ccctcgtggg    120
   gcggcacggg cacgtgcgct cgtctcgctg gggcttggtg tcgatgttcg ctccccctgg    180
   ggtgttcagc gcacctgccc gagtgtgcac ctgtgcgtgt tcgcgttgtg tgtccgcctg    240
   ggcccctgtg gaccaccctg caacggtggg agcgcaggct ctcagcaaac aggtgcagca    300
   gccccgcccc cactcttctg agctgtgtgg ctttgatcag gggacttccc tgtctgttaa    360
   atggaaacct aatagtatct gtagcgagaa gtatggccac ctttatgtga gaaaagggat    420
   tcgcagggct cagcagggct gggcacaagg agagttctgg gctccg                   466
<210> 75
   <211> 454
   <212> DNA
   <213> Homo sapiens
<400> 75
   gcatggcgcc ccaccacggg ctcggtctat ctgcgcgcca agatcccgct tggggcgagg 60
   cgttgggtca gcgtttagag ccactccctg cgctggtggc tggacatagc ctccctatcc 120
   cacctcatct tcccccatcc ccgacagagg aggttgtgaa tctacaggcc cttgacgttg 180
   aggcgtcgga gggcgcacct ttgtaattgc ggcctccctt cgccccttaa gtgccgcttc 240
   tgggcgccta ggctggatat gaaagccccg ttcctaatcc tctgctctgg tcccctcctc 300
   tggactgctg ggactctaag ctaggccctc cccaggttcc atcactgcgg cgccaacccg 360
   cggctgggct gtccgcaaga gggagttgaa ggcgcgcgga atcccgaggt gcagctgacc 420
   ctcctctcaa cgccgactct gccgctcccg cccg 454
<210> 76
   <211> 325
   <212> DNA
   <213> Homo sapiens
<400> 76
   ggacacaggt gcagatctcc agcggagcac tgcggagtgc gcgccgtcga gcactaggga 60
   atcctagacg gaggacttgg tccattccac gcagtcccag gcaggtccgc agcggaggga 120
   cgcagcggtc tccaactcct ggtcacgact tcggcgaccc tccaccccct gagagacctg 180
   gtcccacgga gctgtccccc caggagccgc agcgggaata gcaaagcaaa ggggaccact 240
   cagcccccag gaggagccct gaagcaaaaa ggttgctgcg gggagccacg ttccctctgg 300
   ttcacctcga agcccaggag ctccg 325
<210> 77
   <211> 495
   <212> DNA
   <213> Homo sapiens
<400> 77
   ggacacttaa ttcttcctcc aatccctggt ctctgccgcg tgggctagat ctactgcaag     60
   tgctgggcat ggggaaagga agagagaagt taaaggtgaa atggcttctg ctctcggcct    120
   ctccgtgagc gctctcggcc ctcctgtgga gatcgccacg tcctatcaca cgcgtcccta    180
   aaccttgtca ctcgagcatt cttgtctccc aaactctgat ggtccgcagt gggtactggg    240
   ctccctgggc cctgggagga agacggagga gtttgggtga aagatcagac ggtgcgcagc    300
   cctgaggctt ctgagggcag agggtgcgct tccttgccct cggggcgggg aagccgtgaa    360
   cgccgaggcc attgtaaggc tgggtggtgc ggagcgcggg aagggggctg ggatttgaat    420
   gggagcctgt gattggccga tccctggact gacgtcactt ccccgcgggg cgattagcct    480
   gcgagaggag ggccg                                                     495
<210> 78
   <211> 295
   <212> DNA
   <213> Homo sapiens
<400> 78
   gatcgagggg gacgtcaccc tcggggggct gttccccgtg cacgccaagg gtcccagcgg     60
   agtgccctgc ggcgacatca agagggaaaa cgggatccac aggctggaag cgatgctcta    120
   cgccctggac cagatcaaca gtgatcccaa cctactgccc aacgtgacgc tgggcgcgcg    180
   gatcctggac acttgttcca gggacactta cgcgctcgaa cagtcgctta ctttcgtcca    240
   ggcgctcatc cagaaggaca cctccgacgt gcgctgcacc aacggcgaac cgccg          295
<210> 79
   <211> 211
   <212> DNA
   <213> Homo sapiens
<400> 79
   gatcctggat ccactcggcg gcctcgcagt agaattcgcc ctggtcagaa ggctgcaggt     60
   ggaagatggt gaggcggaag gtggtcctcc ccagcttgtc cagccgcacc tcccccaggc    120
   tctgcctctg ggcatattcg ctgctggagt gaagcatgaa atctcggctc agggagatga    180
   cctccacggg cttctcgcca actttctgcc g                                   211
<210> 80
   <211> 404
   <212> DNA
   <213> Homo sapiens
<400> 80
   gcagcgccat cgagtgagga atccctggag ctctagagcc ccgcgccctg ccacctccct 60
   ggattcttgg gctccaaatc tctttggagc aattctggcc cagggagcaa ttctctttcc 120
   ccttccccac cgcagtcgtc accccgaggt gatctctgct gtcagcgttg atcccctgaa 180
   gctaggcaga ccagaagtaa cagagaagaa acttttcttc ccagacaaga gtttgggcaa 240
   gaagggagaa aagtgaccca gcaggaagaa cttccaattc ggttttgaat gctaaactgg 300
   cggggccccc accttgcact ctcgccgcgc gcttcttggt ccctgagact tcgaacgaag 360
   ttgcgcgaag ttttcaggtg gagcagaggg gcaggtcccg accg 404
<210> 81
   <211> 702
   <212> DNA
   <213> Homo sapiens
<400> 81
   gccctcagaa gatcagctca cagtgggtga gggggaaaga ccagcaggca atgaagacgc 60
   aacgtggccc acctcctccc cgaggacaga cagacccaaa tattgtggat cctactgagg 120
   tccctgagct tctcctccag attcagtcct gatggttggt catcttccac ggctgcttgc 180
   ttccaggcct ctgtacctgc cttccttctt cgcttgccct ccccatcttt ttcttgcatc 240
   cctcactgca ctgggctcta gtgactttca ggtctttatt tccatgccat ctccttgaga 300
   aagacttccc tgcctccctg gtcagctgtc tattttctgt gttccttcag acccctggtt 360
   ttcctgtcac ctatctggcc atgttgcatc ttcattgcct gcttgtctct ccccctcacc 420
   taccatgaac tggtcttctg agggcagggg ctgtgcatcc ttcactctgg agccctcagc 480
   actgactcgg tgcacagtaa gtggctgcca agtggatgga agactgttag cctcttcagg 540
   tccaaggaag aagggtgtat ttggctgggg gctttctttg ggtagacacc aggaaagctt 600
   tctgctttca tatttggctt tatagaagaa cccaggaggc taagctgccc aaagacagtc 660
   ttgcagcagg aactccctct tgatttttca gggtaagctc cg 702
<210> 82
   <211> 462
   <212> DNA
   <213> Homo sapiens
<400> 82
   gattctccta ggacattctt ggtgaaagca aaaaggatgt ccaagcagtg gatcttatct 60
   ccagggacca aaggcaggtc catctggatc agtatatttc gattgggttt tgggattctc 120
   aggggaccag agagagtgtc tgcaaagtcc gagagagcag aaaaggtaat aaactgagtg 180
   gcctctgggt caaacttctc ccaggtctca tagaacatgt caaagtcgtc ctcactcagg 240
   ggctcagtgc tctcctccgt ggccacattg aagttctcca gaatcactgc aatgtacatg 300
   ttgaccatga tgaggaagga gatgatgatg taggtggtga agaagatgat gcctacggct 360
   gggctcccac agtcccctct ggtgccattg ctgttgggca gattggggtc acagtagggg 420
   ggccctgtgt tgaggatggg gctgaggagg ccatcccagc cg 462
<210> 83
   <211> 515
   <212> DNA
   <213> Homo sapiens
<400> 83
   gcctctgctg agaccctgga ggatgcctct ctgctcccag gggaaccatg tcaatggtag 60
   cgctccccca aactcaacac ctgtgagcaa ttcagcctcg ggtggaaaag aaaaaaaaat 120
   gacaaaatga ctgcagcaaa atttaaattt taaaaataat ttgcaaattg gtcattggcc 180
   aaattgattg tatgatgaat tgactggtgt agacatagtt tttggtaaat ttgtttgctt 240
   cctcacaaaa gaactggcaa tccagtttgc aaggatactc accaaggccc cttcccgcct 300
   tgctctggag agggtgttga gaggcagtgg gcaagtggga ggtggggaca ggcgttcagc 360
   caccatcttc cagagttctc attgtgttgc cactggagtc atctgcttct gctttttttt 420
   tttttttttt tttttttttg agatgaagtc tcactctgtc acccaggctg gagtacaaag 480
   gcacgatctt ggctcactgc aacctccgtc tcccg 515
<210> 84
   <211> 485
   <212> DNA
   <213> Homo sapiens
<400> 84
   gagacgctcc ctggaaagga gtgctgatga ggccgctctt gtgtgtgaag gcctggcctg 60
   cagcgccctt ctcccacgtc ccttcccctt gtctgctgcg ctccaagttt gaagccagca 120
   agcataagta ggcgccaagg cagggaggct gtgctgggcc tcaggcgggg ggagctggag 180
   ggaaggaggg agggaggatg ggggaggaaa ggaaggaact gggtggacac agctggaggg 240
   gaggagagag agggaatgtg gggaggcatg gaggcactgc cgagaaaatg gagcaagaaa 300
   gtgaggcggc tcctgcgtgg acccaggcga gcgtgccagg gactgggggc tctggaagat 360
   tcacactgtc agccgtaggc gttctttcct gcccccccag cctcccgcaa cggatgtact 420
   ccgagccctt cgaactctcg gggtaccagt gagaactctg aactgagagt ttcttctgag 480
   atccg 485
<210> 85
   <211> 460
   <212> DNA
   <213> Homo sapiens
<400> 85
   gccaggaggt ggtggcccag gctgggtgag ggacactggg gaagttctaa cataggaagt 60
   cccccaagga gtgaagagat tgcggagggg acacacattc agggtgggtg aggctggacg 120
   tgcagtcctg ctggttttct gggttgggca tgtgattgat aaaagtggag ttgagggcac 180
   attggcactg ggagtcaaat gcgcagaaga cttgagcttg ctactctccc gagccaggag 240
   ctctaaggtg ttttcattgt tgaaatccaa gggttctgat ttaggcttgg ctggcacgtt 300
   gcaagtgacc ccatacttgt ggaatcaact gaggctgatg gattctgcaa agcctctgac 360
   acctcctgat tttggtggtc ctgagcatga agtgagcagg gaatacatgt ggggataaac 420
   gtggcaaagt gccctgttac ccccatccaa agacttcccg 460
<210> 86
   <211> 350
   <212> DNA
   <213> Homo sapiens
<400> 86
   gacgccgacc cgcgcccctt gtcaccctgt acacacactc gaaccctcaa acacccacct     60
   cctcttcccc caaaacatcc agtgggcgct cttcacgccc cttcaggcga catagaccca    120
   gctgacaacg ggacacacat gtcccctcat gtcctcgtcc tctccgccaa ggacgggtgc    180
   gtcctcagaa ctcgctgaag aggtgtgaaa gttttgtttg ggttttgcgc gcgagatgaa    240
   actctataag catttactcc tacctgacac gcactgcgtg gggaggaaga aaagtggcgg    300
   tggatgaagt gaacccacct tcgcggagta gaacccacag ttatattccg               350
<210> 87
   <211> 240
   <212> DNA
   <213> Homo sapiens
<400> 87
   ggttcagacc tgagtgttca aaatcccaaa tatcaacctc ttaaccactg cctcttggat     60
   ccttcaggag gaagagcaac tgggaaccca caactgaccg ctattacctg atccccagtc    120
   ctagcctggg cggaaagttt catataactt gagacggtgc gcgtggggaa aacgtaaagc    180
   tcccgttcag ggctgctcct gcgcggaagc tgcgtgggct aggagaagga ggctccgccg    240
<210> 88
   <211> 408
   <212> DNA
   <213> Homo sapiens
<400> 88
   gcccccagca gcgcccatct ccactccacg caccaggctc ctctcacctg actctcccgt     60
   ggaaactcgt ggcgcacgat gctgatcact ggaatgtgca ggactaagct gaccaagtcg    120
   agctccatca tttcgccctg gctctggggg aaggcgagca gcgccgacac cccttgcacc    180
   accacggtat ggcacacact ttgcaggaag gagaaagggt cactgctcca tggcgaacta    240
   ggggaggaga agggcaaaag tggcagatcg cccaggcctg cctcgatggc catcactact    300
   tccaaagaca ggttgtaggg tagcagccct tccacgcggt tcaggttgtc cacggcaaat    360
   aggagggcgt cccgtggcca cagggcctcc gccctggcgc cctccccg                 408
<210> 89
   <211> 75
   <212> DNA
   <213> Homo sapiens
<400> 89
   gggctgccac aaacgccacg acttggcttg gcctctctct tagttattcg cagctcagcc 60
   cgatgggcgt ctccg 75
<210> 90
   <211> 535
   <212> DNA
   <213> Homo sapiens
<400> 90
   ggtccccctc gaagtcaaaa tcagccatgt gagcagtctt tcccgactca gagaccccaa 60
   atcctcacca cacattagca ggatattgat cattcactga acacattggg gcaagacagg 120
   gatccacgca taatggaaat aatttcataa aatccatttt aaaactggtc aatcccgtct 180
   cgagttcctc tccagctgcc aggctggtcc ttcccatctt tcagcctcca cacccatcac 240
   tccactcagc taaaacaccc cctctaccac ctagacctgc cctccctgga aaggagaggt 300
   tatacttaat gaatctccct gtttttcaaa atatcagaca gtaagggact ttattccttc 360
   cttcttccct gtctcccata catttccttc ctttcacttc ctcccttccc catgtgacac 420
   tcaccaagca ccaagaagat cttcctccac ccagaggcaa tgaagacatc ccaggcaaca 480
   gccctaggca gaccacagag ggtagccagg ctcgaaggct tcagacaaag agccg 535
<210> 91
   <211> 103
   <212> DNA
   <213> Homo sapiens
<400> 91
   gagcaattga aatatcccca tcctgagcgg tctcttttct aggatcaaga tgaacacact 60
   gcagacgagg acacgagccc cacaggagct ctttgtcccg ccg 103
<210> 92
   <211> 414
   <212> DNA
   <213> Homo sapiens
<400> 92
   gcaagcctct ggctgcagcc tggagtctag agcgaacctg ctaacacatc ttgcagcaag 60
   agcaggagta gcaaagaagg gcacaaaaaa taaagaatgg catggctcct cccttggacg 120
   caggcaccca ggggcacctc cccgacactc cccattcccc agaggggccc ttgggaatga 180
   ggcccagcac ccctcacttc tcctttgagc aaccatctca tgaggaaggc gaggcagggg 240
   ccaatagaac ccttgtacag acggagaaaa cgaggaccag agagggaatg agacttgccc 300
   caaatccacc caaggtgaca gcaggagccc agatctttgg ctttccagac acaataacag 360
   caacagcacc agcaaccatt aactgggggg tacgttctgg gccccacaca cccg 414
<210> 93
   <211> 320
   <212> DNA
   <213> Homo sapiens
<400> 93
   gtccagaagc tgagagtcac gcggtggcag gccagcggca ggagccagac agcagccaga 60
   gctagggccc tctagggctg gaagcctctc cctctcggtt ttcacatggc tggggaattt 120
   ggctctgctc tatttccttt ctgaaatgtt cgcttcagca ggtttaaagt ccagcggatg 180
   gggaacgttc catgctggct gtgagcggcc tctttctggc ctggatgatt ttttctggtg 240
   tgaatcccat gcacgtggag agcattggtg tctaccatct ccatgaaatg caggaaggca 300
   gcttctgggt tgagctgccg 320
<210> 94
   <211> 366
   <212> DNA
   <213> Homo sapiens
<400> 94
   ggccacatcc cctgccccgt tgtgccttcc acacctcggg cagtcactag gaaaagggtc 60
   gccaactgaa aggcctgcag gaaccaggat gatacctgcg tcagtcccgc ggctgctgcg 120
   agtgcgcgct ctcctgccag ggggacctca gaccctcctt tacagcacac cgagggccct 180
   gcagacacgc gagcgggcct tcagtttgca aaccctgaaa gcgggcgcgg tccaccagga 240
   cgatctggca gggctctggg tgaggaggcc gcgtctttat ttggggtcct cgggcagcca 300
   cgttgcagct ctgggggaag actgcttaag gaacccgctc tgaactgcgc gctggtgtcc 360
   tctccg 366
<210> 95
   <211> 302
   <212> DNA
   <213> Homo sapiens
<400> 95
   gaccccgaag cggttgaggg gatccaggcc tcacaacgtc ttgagagtgg ccccagcgtc     60
   gttcctagtc tcttcggcag accccagcgt cgtccttctt ttcctgagaa tatcctgaca    120
   ccagtcgccc gttccagaag gggacacttg gcgtcatccc tttttttctg agtgacccca    180
   gagtcgtcct cacttcctga gggggatcct ggcgtcatcc gcccttcttg aggggaccct   240
   gctgtcacta ccctgtcctg atggccagca ccgcattgtc ctttcttccc gcgggaaacc    300
   cg                                                                   302
<210> 96
   <211> 1067
   <212> DNA
   <213> Homo sapiens
<400> 96
   gccaacactt agggaaaata gaaagaacct acgctgaaat attgggggct ggttcttctg     60
   atacccaaca ccatggctca cagctgtaat tccaacactt ccagaggcca aggcaggagg    120
   atcacttgaa ccttggagtt tgaggttgcc atgagctatg attgtaccag tgcactccga    180
   cctgggtgac acagtgagac cttgtctcta ataagaagaa aaaaatgcca ttttaaaagt    240
   gtacactttc atggttttta gtatattcac tgtattgtac aaccattacc actacctcat    300
   tccagaacac ttccaccacc cccaaaagaa agcagttaca tctgttcact gtagtgccaa    360
   ttactcaacc ccttgtcatc ccctggcaac caccactcag gtgtactttc tatgtctgtg    420
   ggtttgctat tgtaatttga aagttcactg atgttacttt aatttggttt cattttgaat    480
   acagtaaata tggatcaaaa cccatatata cggagcatct ttagaggcct catttttgtt    540
   tttttttttt tttttgagat ggagtcttgc actgttgccc aggctggagt gcagtggcgt    600
   gatctcggct cactgcaaca tccacctccc agattcaagc gattctcctg ccccagcctc    660
   cccagtagct gggaatacag gcgcctgcca ccacaccctg ctaatttttt tatttttagt    720
   agagaggggt tttcactatg ttggccaggc tggtcttgaa cgcctgacct cgtgatgcac    780
   ctgcctcggc ctcccaaagt gctgggatta caggcgtgag ccaccacgcc caggctagag    840
   gcctcatttt ttaagactaa tgggatccca aggctaaacg tttgagaaac actgatctaa    900
   gatttgtgtt ttgttttgtt ttgttgttca atcaagtgga acataatgtc taaaccaggg    960
   ccttacctac ctggaactca aaggtggaat gaccttccct ggccaagctt ccagaaggga   1020
   cagggagcta cttaacagac tgctggaccc ttaacgactg gaaaccg                 1067
<210> 97
   <211> 259
   <212> DNA
   <213> Homo sapiens
<400> 97
   gggcttccat ggtttcaggt tttccttccc ttcctttttc cccaaggtcg ctggaaccag     60
   ggctgccttc cagcacttca tggggcacct ggtacttctg gccgtgtggc caaaggcccc    120
   gcagtttttg cacttgagct gtgggtggaa aggaagtgat gtcagtgagt gagctgaagc    180
   cacaggcagc gatcccacgt caacattggg acggattgtg aattcagagc tgaataagga    240
   ttccaaagag gggacaccg                                                  259
<210> 98
   <211> 346
   <212> DNA
   <213> Homo sapiens
<400> 98
   gattcgccct cggacgcccc agctgggcac actcacgcgg ctctcccgcg gcttctcggg     60
   aattcgcctc cagggcaatc agctcttcgc acaaacgttc aaaccaaggt aagagctatc    120
   agatccagcc agatctgctt cctaagcctg gccagggccc taagcacccc cacccacacg    180
   ctcagaggcg ggcgacgccg aggcagcgcg aacccgcccc acgcggtcag ctccaaggcg    240
   ccctgtttag cgttacctct gcgaccctgg gatgggcctg ggcaggggct ggcttcccct    300
   ggctaaacgc agtcaatgaa gggggttcac tttcctaaat acgccg                   346
<210> 99
   <211> 426
   <212> DNA
   <213> Homo sapiens
<400> 99
   gggactctgc agcctgcaga cagcagcaga cccaacgcct agagatttac cgtgcgcgcc     60
   cctgaagtta cccagcgccc cgcaggccac cgcgcccctc gcggctcgca ggcctggctg    120
   gacgccgcag gcctggctgg atgccgcagc ccttcacaga gagcaggctc ctccgcgcac    180
   gactgcagcc ccagtcgttt gcagttccct tgccatttat gggacccttt ggcttttaca    240
   gagcgtgtcc agccatcaga gtgcggaagc cagtgcgcag gtcactaaca tttacaaagc    300
   acaggccctt gacagtttat actacccgtg gaggtctcaa gcggcagggc ccctctcccc    360
   tgttccctgt ccctgaggtg ggggaatggg gatggggagc ggccgccccc ccctcccccg    420
   aggccg                                                                426
<210> 100
   <211> 838
   <212> DNA
   <213> Homo sapiens
<400> 100
   gtgtgctctg agcccgccag acaacacctg gatctcagcc ccacaaatgg acacagaaga     60
   tgtatcacca aaacaaatcc atctttggtc tctgtccctg gttcctggca cagaactgct    120
   aaaaccctta aaatttcctg aatgtcagaa gtactaagca catccctggt tctaatattt    180
   ggtctttgac cccagtatct gacacagagc tcctaaaccc tttgggagga taggagcatc    240
   ttttgttcta atgagcaatt cttggtgggc tcctgggtgg gggctggtcg ccagaaagat    300
   caagccagga ttagaagctt ggaactttca agcttccacg cccatcctcg gggaggggag    360
   aagggctgga gattgagtta ataatagaga atgccagcga ggtgcatcac actcacgcct    420
   gtaatgccag cactttggga ggctaacatg ggaagaccac ttgagcccag gagtttgaga    480
   ccagcctgga caacctagta aaacccatct ctacaaaaaa atggaaactt atctaggcat    540
   gatggcacat gcctgcagac ccagggactg gggaggctga aggaggaaga ttgtttgagc    600
   cccagaggtc aaggctgcac caagttgtga ctgcaccact gtgctccagc ctggaccaca    660
   gagagaccct gtcccactcc aagaaaataa tccatcatgc ctaagcctca caaaatccct    720
   agaagacgag gtttggagag cctctgggtt ggtgagcatt cccaccagcc agcaaggtgg    780
   gagaccccaa ctccacgggg gagaccccaa ctccacaggg aagaagctcc tgtgcccg      838

## Description

### FIELD OF THE INVENTION

The present invention is in the field of biology and chemistry, more particularly in the field of molecular biology and human genetics. The invention relates to the field of identifying methylated sites in human DNA, in particular methylated sites in certain defined sequences which when methylated are indicative of breast cancer.

### BACKGROUND OF THE INVENTION

Worldwide, breast cancer is the fifth most common cause of cancer death (after lung cancer, stomach cancer, liver cancer, and colon cancer). In 2005, breast cancer caused 502,000 deaths (7% of cancer deaths; almost 1% of all deaths) worldwide. Among women worldwide, breast cancer is the most common cancer and the most common cause of cancer death.

In the United States, breast cancer is the third most common cause of cancer death (after lung cancer and colon cancer). In 2007, breast cancer is expected to cause 40,910 deaths (7% of cancer deaths; almost 2% of all deaths) in the U.S. Among women in the U.S., breast cancer is the most common cancer and the second most common cause of cancer death (after lung cancer). Women in the U.S. have a 1 in 8 lifetime risk of developing invasive breast cancer and a 1 in 33 risk of breast cancer causing their death.

Breast cancer is diagnosed by the pathological (microscopic) examination of surgically removed breast tissue. A number of procedures can obtain tissue or cells prior to definitive treatment for histological or cytological examination. Such procedures include fine-needle aspiration, nipple aspirates, ductal lavage, core needle biopsy, and local surgical excision biopsy. These diagnostic steps, when coupled with radiographic imaging, are usually accurate in diagnosing a breast lesion as cancer. Occasionally, pre-surgical procedures such as fine needle aspirate may not yield enough tissue to make a diagnosis, or may miss the cancer entirely. Imaging tests are sometimes used to detect metastasis and include chest X-ray, bone scan, CT, MRI, and PET scanning. While imaging studies are useful in determining the presence of metastatic disease, they are not in and of themselves diagnostic of cancer. Only microscopic evaluation of a biopsy specimen can yield a cancer diagnosis. Ca 15.3 (carbohydrate antigen 15.3, epithelial mucin) is a tumor marker determined in blood which can be used to follow disease activity over time after definitive treatment. Blood tumor marker testing is not routinely performed for the screening of breast cancer, and has poor performance characteristics for this purpose.

Therefore, it would advantageous to have a method for the analysis of breast cancer disorders which is quick, reliable and can ideally be performed by untrained personal. Such a method would ideally not require an analysis by a trained physician.

WO 2005/123945, WO 2006/008128, WO 2005/059172 and WO 2007/019670 disclose the analysis and detection of breast cancer by analysing the methylation of at least one gene promoter of numerous genes.

### SUMMARY OF THE INVENTION

The present invention relates to a method for the analysis of breast cancer disorders, comprising determining in a panel of DNA sequences the genomic methylation status of one or more CpG dinucleotides in each sequence, wherein the DNA sequences are selected from the group of SEQ ID NO. 1 to SEQ ID NO. 100, in particular from the group of sequences according to SEQ ID NO. 1 to SEQ ID NO. 10 and SEQ ID NO. 50 to SEQ ID NO. 60.

The regions of interest are designated in table 1A and table 1B ("start" and "end").

CpG islands are regions where there are a large number of cytosine and guanine adjacent to each other in the backbone of the DNA (*i.e.* linked by phosphodiester bonds). They are in and near approximately 40% of promoters of mammalian genes (about 70% in human promoters). The "p" in CpG notation refers to the phosphodiester bond between the cytosine and the guanine.

The length of a CpG island is typically 300-3000 base pairs. These regions are characterized by CpG dinucleotide content equal to or greater than what would be statistically expected (≈6%), whereas the rest of the genome has much lower CpG frequency (≈1%), a phenomenon called CG suppression. Unlike CpG sites in the coding region of a gene, in most instances, the CpG sites in the CpG islands of promoters are unmethylated if genes are expressed. This observation led to the speculation that methylation of CpG sites in the promoter of a gene may inhibit the expression of a gene. Methylation is central to imprinting alongside histone modifications. The usual formal definition of a CpG island is a region with at least 200 bp and with a GC percentage that is greater than 50% and with an observed/expected CpG ratio that is greater than 0.6.

Herein, a CpG dinucleotide is a CpG dinucleotide which may be found in methylated and unmethylated status *in vivo,* in particular in human.

The invention relates to a method, wherein a primary cancer is detected using the methylation pattern of one or more sequences disclosed herein and also, wherein the methylation pattern obtained is used to predict the therapeutic response to a treatment of a breast cancer.

Herein, a subject is understood to be all persons, patients, animals, irrespective whether or not they exhibit pathological changes. In the meaning of the invention, any sample collected from cells, tissues, organs, organisms or the like can be a sample of a patient to be diagnosed. In a preferred embodiment the patient according to the invention is a human. In a further preferred embodiment of the invention the patient is a human suspected to have a disease selected from the group of, primary breast cancer, secondary breast cancer, surface epithelial-stromal tumor, sex cord-stromal tumor, germ cell tumor.

The method is for use in the improved diagnosis, treatment and monitoring of breast cell proliferative disorders, for example by enabling the improved identification of and differentiation between subclasses of said disorder and the genetic predisposition to said disorders. The invention presents improvements over the state of the art in that it enables a highly specific classification of breast cell proliferative disorders, thereby allowing for improved and informed treatment of patients.

Herein, the sequences claimed also encompass the sequences which are reverse complement to the sequences designated.

### BRIEF DESCRIPTION OF THE DRAWINGS

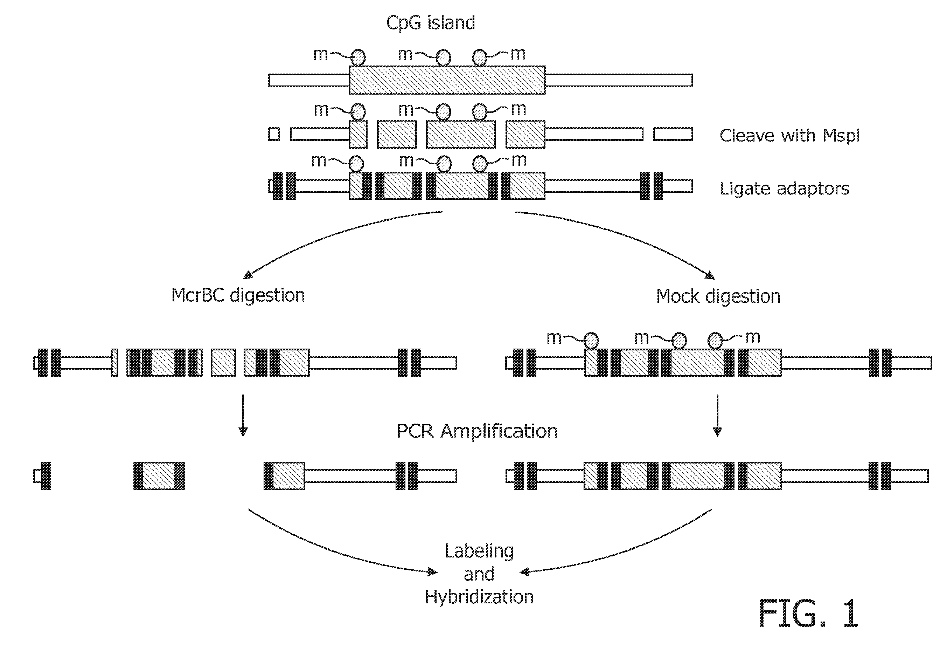

- Fig. 1: shows the method for determination of differentially methylated regions of the genome. This is outlined in more detail in the Examples.

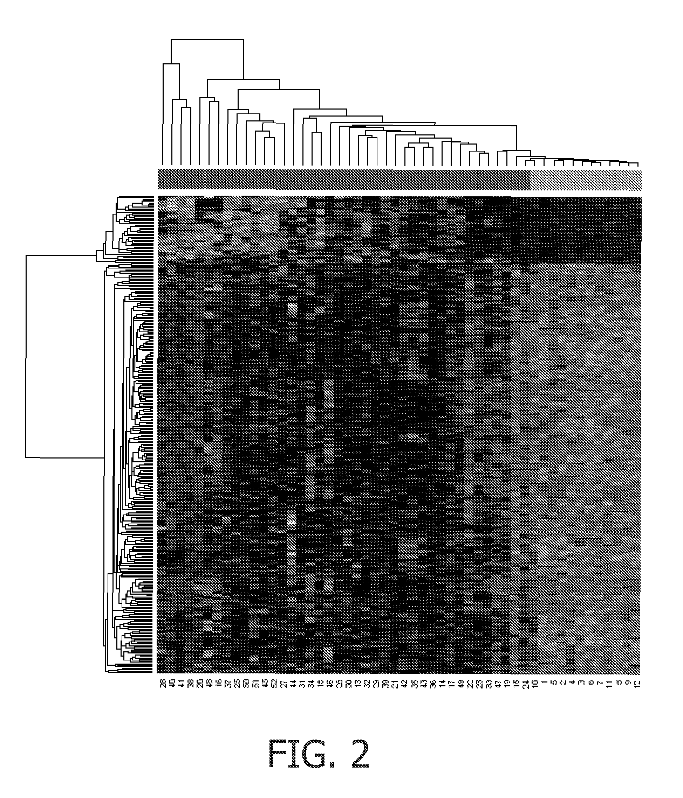

- Fig. 2: shows clustered samples (columns) vs. methylation loci (rows). Methylation signatures can differentiate between tumors (left part of bar on top) and normal tissue (right part of bar on top).

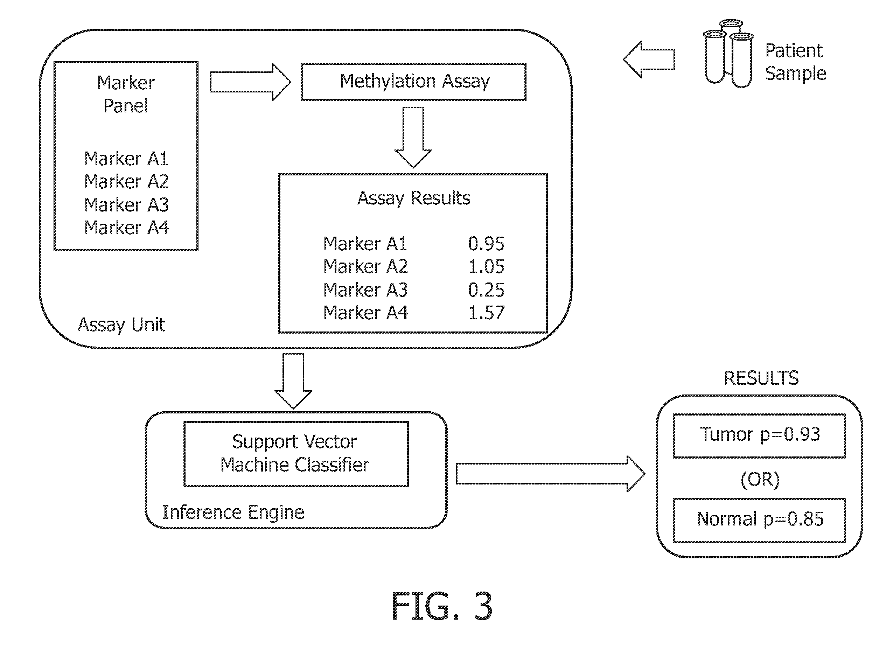

- Fig. 3: shows a clustering the method for building the invention and its salient features. A sample of the patient is collected and the methylation status of specific sequences is determined by any one of the preferred embodiments. Then, the results are fed into a classifier such as support vector machine which provides a classification as a tumor sample or normal sample and p-value.

### DETAILED DESCRIPTION OF EMBODIMENTS

The inventors have astonishingly found that a small selection of DNA sequences may be used to analyze breast cancer disorders. This is done by determining genomic methylation status of one or more CpG dinucleotides in any of the sequences disclosed herein or its reverse complement. About 900 sequences were identified in total that are suited for such an analysis. It turns out that 100 sequences are particularly suited.

Based on just 10 sequences, such as the top ten features from Table 1A or 1B (p-value 0.000.1), it is possible to arrive at a classification accuracy for of 94% (Total correct predictions with respect to the question of whether a given sample is from an breast tumor or not versus total predictions performed, 49/52). The sensitivity for tumor detection was 92.5% (37/40), the specificity for tumor detection was 100%). Increasing the feature size to 50 gives a classification rate of 96% (50/52 classified correctly).

The sequences may be found in genes as can be seen in table 1A below.

**Table 1A**

| SEQ ID NO. | ID | Chromosome | Start | End | P-val | Gene_name |
|---|---|---|---|---|---|---|
| 1 | ID173583 | chr8 | 125810238 | 125810819 | 0.0000217 | MTSS1 |
| 2 | ID135122 | chr4 | 9040795 | 9041453 | 0.000058 | DUB3 |
| 3 | ID59231 | chr15 | 87711410 | 87711904 | 0.0000000747 | hsa-mir-9-3 |
| 4 | ID135160 | chr4 | 9459627 | 9459776 | 0.0000000115 | DRD5 |
| 5 | ID123222 | chr22 | 43445548 | 43445907 | 0.000000192 | PRR5 |
| 6 | ID41349 | chr12 | 105476974 | 105477298 | 0.000000362 | RFX4 |
| 7 | ID146518 | chr5 | 140703634 | 140703867 | 0.000000443 | PCDHGA3 |
| 8 | ID66687 | chr16 | 65169983 | 65170374 | 0.000000574 | AY862139 |
| 9 | ID11596 | chr1 | 146066973 | 146067308 | 0.000000872 | AK123662 |
| 10 | ID112724 | chr20 | 22514937 | 22515431 | 0.0000012 | FOXA2 |
| 11 | ID56406 | chr15 | 50874380 | 50874668 | 0.00000131 | ONECUT1 |
| 12 | ID11658 | chr1 | 146486000 | 146486341 | 0.00000157 | AK123662 |
| 13 | ID114005 | chr20 | 39198472 | 39198934 | 0.00000162 | PLCG1 |
| 14 | ID41387 | chr12 | 105851242 | 105851742 | 0.00000276 | MGC17943 |
| 15 | ID130737 | chr3 | 138963266 | 138963653 | 0.0000029 | SOX14 |
| 16 | ID27050 | chr11 | 3819507 | 3820119 | 0.00000306 | RHOG |
| 17 | ID98568 | chr19 | 63407518 | 63407732 | 0.00000467 | ZNF274 |
| 18 | ID160851 | chr7 | 35070796 | 35071213 | 0.0000062 | TBX20 |
| 19 | ID9698 | chr1 | 92126308 | 92126790 | 0.00000624 | BRDT |
| 20 | ID35001 | chr11 | 124134059 | 124134403 | 0.0000129 | AY189281 |
| 21 | ID188098 | chrX | 113641444 | 113641884 | 0.0000135 | BC028688 |
| 22 | ID41218 | chr12 | 103034912 | 103035336 | 0.0000139 | NFYB |
| 23 | ID4450 | chr1 | 23416592 | 23417362 | 0.0000151 | HNRPR |
| 24 | ID97179 | chr19 | 56695742 | 56696075 | 0.0000166 | SIGLEC12 |
| 25 | ID137603 | chr4 | 89285337 | 89285745 | 0.0000208 | PKD2 |
| 26 | ID77777 | chr17 | 55854004 | 55854719 | 0.0000242 | LOC124773 |
| 27 | ID146531 | chr5 | 140715120 | 140715429 | 0.0000303 | PCDHGB2 |
| 28 | ID76724 | chr17 | 44159203 | 44159574 | 0.0000679 | PRAC |
| 29 | ID135120 | chr4 | 9006410 | 9006713 | 0.0000874 | DUB3 |
| 30 | ID135121 | chr4 | 9017069 | 9017727 | 0.0000911 | AY509884 |
| 31 | ID71929 | chr17 | 7695823 | 7696284 | 0.000130076 | LOC92162 |
| 32 | ID11593 | chr1 | 146066575 | 146066841 | 0.000154186 | AK123662 |
| 33 | ID120446 | chr22 | 20546877 | 20547317 | 0.0001669 | MAPK1 |
| 34 | ID146484 | chr5 | 140601695 | 140601937 | 0.000192752 | PCDHB15 |
| 35 | ID103546 | chr2 | 86334450 | 86334476 | 0.000264959 | MRPL35 |
| 36 | ID161220 | chr7 | 43853299 | 43853383 | 0.00030013 | DBNL |
| 37 | ID11654 | chr1 | 146485690 | 146485868 | 0.000310651 | AK123662 |
| 38 | ID146595 | chr5 | 140777723 | 140778009 | 0.000318226 | PCDHGA11 |
| 39 | ID173389 | chr8 | 121206506 | 121207025 | 0.000396103 | COL14A1 |
| 40 | ID160133 | chr7 | 26919212 | 26919376 | 0.000432818 | HOXA2 |
| 41 | ID118279 | chr21 | 42946007 | 42946287 | 0.000498108 | PDE9A |
| 42 | ID68965 | chr16 | 86694584 | 86695293 | 0.000498402 | AK126852 |
| 43 | ID16024 | chr1 | 224770811 | 224771150 | 0.000548832 | BC043916 |
| 44 | ID91933 | chr19 | 18831977 | 18832267 | 0.000607126 | AK125797 |
| 45 | ID146581 | chr5 | 140768066 | 140768556 | 0.000680057 | PCDHGA10 |
| 46 | ID61023 | chr16 | 954593 | 954879 | 0.000792141 | AK127296 |
| 47 | ID146570 | chr5 | 140757958 | 140758452 | 0.000996446 | PCDHGA9 |
| 48 | ID171504 | chr8 | 65454257 | 65455748 | 0.000953039 | hsa-mir-124a-2 |
| 49 | ID168737 | chr8 | 9798186 | 9798550 | 0.000137444 | hsa-mir-124a-1 |
| 50 | ID12521 | chr1 | 153203369 | 153203671 | 0.000101994 | hsa-mir-9-1 |

The sequences may be found in intergenic regions as can be seen in Table 1B.

**Table 1B**

| SEQ ID NO. | ID | Chromosome | Start | End | P-val |
|---|---|---|---|---|---|
| 51 | ID33426 | chr11 | 89160048 | 89160322 | 1.14E-10 |
| 52 | ID90896 | chr19 | 15148984 | 15149357 | 1.14E-10 |
| 53 | ID29499 | chr11 | 49026728 | 49027002 | 4.06E-10 |
| 54 | ID169777 | chr8 | 24827761 | 24828171 | 6.48E-10 |
| 55 | ID109204 | chr2 | 220021958 | 220022344 | 8.65E-10 |
| 56 | ID103749 | chr2 | 91295935 | 91296161 | 1.82E-09 |
| 57 | ID99161 | chr2 | 2812784 | 2813304 | 1.82E-09 |
| 58 | ID45297 | chr13 | 27400198 | 27400742 | 3.38E-09 |
| 59 | ID166666 | chr7 | 149354316 | 149354562 | 6.06E-09 |
| 60 | ID167174 | chr7 | 152884159 | 152884405 | 6.96E-09 |
| 61 | ID34211 | chr11 | 113989177 | 113989682 | 9.11E-09 |
| 62 | ID152478 | chr6 | 42253517 | 42253868 | 9.63E-09 |
| 63 | ID24712 | chr10 | 130382122 | 130382412 | 9.63E-09 |
| 64 | ID49246 | chr14 | 28324500 | 28324758 | 1.30E-08 |
| 65 | ID34960 | chr11 | 123811259 | 123816128 | 1.34E-08 |
| 66 | ID112713 | chr20 | 22506327 | 22506681 | 1.59E-08 |
| 67 | ID54570 | chr15 | 24795835 | 24796140 | 2.70E-08 |
| 68 | ID89508 | chr19 | 9469673 | 9470021 | 2.98E-08 |
| 69 | ID13622 | chr1 | 177614171 | 177614509 | 3.10E-08 |
| 70 | ID1820 | chr1 | 3672455 | 3672910 | 3.10E-08 |
| 71 | ID29015 | chr11 | 45136542 | 45137024 | 3.10E-08 |
| 72 | ID91861 | chr19 | 18622132 | 18622478 | 3.46E-08 |
| 73 | ID77745 | chr17 | 55571595 | 55571965 | 4.18E-08 |
| 74 | ID98238 | chr19 | 61846590 | 61847055 | 4.44E-08 |
| 75 | ID76689 | chr17 | 44074514 | 44074967 | 4.50E-08 |
| 76 | ID76692 | chr17 | 44075076 | 44075400 | 6.20E-08 |
| 77 | ID59231 | chr15 | 87711410 | 87711904 | 7.47E-08 |
| 78 | ID124608 | chr3 | 6878205 | 6878499 | 8.97E-08 |
| 79 | ID10950 | chr1 | 116868496 | 116868706 | 9.58E-08 |
| 80 | ID159953 | chr7 | 24097508 | 24097911 | 9.58E-08 |
| 81 | ID115475 | chr20 | 58536847 | 58537548 | 1.23E-07 |
| 82 | ID126115 | chr3 | 38714269 | 38714730 | 1.92E-07 |
| 83 | ID71392 | chr17 | 6057091 | 6057605 | 2.51E-07 |
| 84 | ID105601 | chr2 | 121341432 | 121341916 | 2.61E-07 |
| 85 | ID168382 | chr8 | 1982797 | 1983256 | 2.61E-07 |
| 86 | ID147288 | chr5 | 158456751 | 158457100 | 2.69E-07 |
| 87 | ID137304 | chr4 | 81466911 | 81467150 | 3.30E-07 |
| 88 | ID179567 | chr9 | 101579069 | 101579476 | 3.92E-07 |
| 89 | ID3487 | chr1 | 16606704 | 16606778 | 4.25E-07 |
| 90 | ID92361 | chr19 | 34425570 | 34426104 | 4.25E-07 |
| 91 | ID177598 | chr9 | 66276397 | 66276499 | 4.45E-07 |
| 92 | ID3846 | chr1 | 18994906 | 18995319 | 5.42E-07 |
| 93 | ID35773 | chr12 | 125067 | 125386 | 5.88E-07 |
| 94 | ID117488 | chr21 | 33327565 | 33327930 | 7.29E-07 |
| 95 | ID89802 | chr19 | 10450948 | 10451249 | 8.72E-07 |
| 96 | ID64615 | chr16 | 29702807 | 29703873 | 8.92E-07 |
| 97 | ID168612 | chr8 | 7917174 | 7917432 | 9.20E-07 |
| 98 | ID16187 | chr1 | 225850241 | 225850586 | 9.73E-07 |
| 99 | ID73339 | chr17 | 21160807 | 21161232 | 1.13E-06 |
| 100 | ID18029 | chr10 | 11462206 | 11463043 | 1.53E-06 |

The genes that form the basis of the present invention are preferably to be used to form a "gene panel", *i.e.* a collection comprising the particular genetic sequences of the present invention and/or their respective informative methylation sites. The formation of gene panels allows for a quick and specific analysis of specific aspects of breast cancer. The gene panel(s) as described and employed in this invention can be used with surprisingly high efficiency for the diagnosis, treatment and monitoring of and the analysis also of a predisposition to breast cell proliferative disorders in particular however for the detection of breast tumor.

In addition, the use of multiple CpG sites from a diverse array of genes allows for a relatively high degree of sensitivity and specificity in comparison to single gene diagnostic and detection tools.

The invention relates to a method for the analysis of breast cancer disorders, comprising determining the genomic methylation status of one or more CpG dinucleotides in each sequence, of the group of sequences according to SEQ ID NO. 1 to SEQ ID NO. 10 and/or SEQ ID NO. 51 to SEQ ID NO. 60. The methylation status of one or more of the sequences according to SEQ ID NO. 1 to 100 may be determined, wherein the sequence has a p-value as determined herein which is smaller than 1E-4 (0.0001) as designated in table 1A or 1B.

In one embodiment of the method according to the invention, the analysis is detection of breast cancer in a subject and wherein the following steps are performed: (a) providing a sample from a subject to be analyzed, (b) determining the genomic methylation status of one or more CpG dinucleotides in each sequence, of the group of sequences according to SEQ ID NO. 1 to SEQ ID NO. 10 and/or SEQ ID NO. 51 to SEQ ID NO. 60.

The methylation status of CpG islands is indicative for breast cancer. Preferably, however, the methylation status is determined for each CpG and the differential methylation pattern is determined, because not all CpG islands necessarily need to be methylated.

Optionally, the following additional steps are performed, (a) the one or more results from the methylation status test is/are input into a classifier that is obtained from a Diagnostic Multi Variate Model, (b) the likelihood is calculated as to whether the sample is from a normal tissue or an breast cancer tissue, and/or (c) an associated p-value for the confidence in the prediction is calculated.

For example, we use a support vector machine classifier for "learning" the important features of a tumor or normal sample based on a pre-defined set of tissues from patients. The algorithm now outputs a classifier (an equation in which the variables are the methylation ratios from the set of features used). Methylation ratios from a new patient sample are then put into this classifier. The result can be 1 or 0. The distance from the marginal plane is used to provide the p-value.

It is preferred that additionally the genomic methylation status is determined for one or more of the DNA sequences selected from the group of sequences according to SEQ ID NO. 11 to SEQ ID NO. 49 and SEQ ID NO. 61 to SEQ ID NO. 100.

The methylation status may be determined for at least ten DNA sequences, at least twenty sequences, at least thirty sequences, at least forty sequences or more than fourty sequences of the sequences according to SEQ ID. NO. 1 to SEQ ID NO. 100. It is particularly preferred that the methylation status is determined for all of the sequences according to SEQ ID NO. 1 to SEQ ID NO. 100.

In one embodiment, the methylation status is determined for the DNA sequences according to SEQ ID. NO. 1 to SEQ ID NO. 10 and SEQ ID NO. 51 to SEQ ID NO. 60. In principle, the disclosure also relates to determining the methylation status of only one of the sequences according to SEQ ID NO. 1 to SEQ ID NO. 100.

There are numerous methods for determining the methylation status of a DNA molecule. It is preferred that the methylation status is determined by means of one or more of the methods selected form the group of bisulfite sequencing, pyrosequencing, methylation-sensitive single-strand conformation analysis(MS-SSCA), high resolution melting analysis (HRM), methylation-sensitive single nucleotide primer extension (MS-SnuPE), base-specific cleavage/MALDI-TOF, methylation-specific PCR (MSP), microarray-based methods, and MspI cleavage. An overview of the further known methods of detecting 5-methylcytosine may be gathered from the following review article: Rein, T., DePamphilis, M. L., Zorbas, H., Nucleic Acids Res. 1998, 26, 2255. Further methods are disclosed in US 2006/0292564A1.

In a preferred embodiment, the methylation status is determined by MspI cleavage, ligation of adaptors, McrBC digestion, PCR amplification, labeling and subsequent hybridization.

It is preferred that the sample to be analyzed is from a tissue type selected from the group of tissues such as, a tissue biopsy from the tissue to be analyzed, vaginal tissue, tongue, pancreas, liver, spleen, ovary, muscle, joint tissue, neural tissue, gastrointestinal tissue, tumor tissue, body fluids, blood, serum, saliva, and urine.

In a preferred embodiment, a primary breast cancer is detected.

The present disclosure also relates to probes, such as oligonucleotides which are in the region of up CpG sites. The oligomers according to the present invention are normally used in so called "sets" which contain at least one oligonucleotide for each of the CpG dinucleotides within SEQ ID NO. 1 through SEQ ID NO. 100 or at least for 10, preferred, 20, more preferred 30 most preferred more than 50 of said sequences. The disclosure also relates to the reverse complement of the oligonucleotides which are in the region of the CpG sites.

The probes to be used for such analysis are defined based on one or more of the following criteria: (1) probe sequence occurs only once in the human genome; (2) probe density of C/G nucleotides is between 30% and 70%; (3) melting characteristics of hybridization and other criteria are according to Mei R et al. Proc. Natl. Acad. Sci. USA, 2003, Sept. 30; 100(20).11237-42.

In a very preferred embodiment, the invention relates to a set of oligonucleotides, for the detection of the cytosine methylation state of genomic DNA according to SEQ ID NO. 1 to SEQ ID NO. 10 or sequences complementary thereto, wherein the set comprises probe molecules specific for the sequences according to SEQ ID NO. 1 to 10. The oligonucleotide according to the invention may be specific for the sequence as it occurs *in vivo* or it may be specific for a sequence which has been bisulfite treated. Such a probe is between 10 and 80 nucleotides long, more preferred between 15 and 40 nucleotides long.

In the case of the sets of oligonucleotides according to the present invention, it is preferred that at least one oligonucleotide is bound to a solid phase. It is further preferred that all the oligonucleotides of one set are bound to a solid phase.

The present invention further relates to a set of at least 10 probes (oligonucleotides and/or PNA-oligomers) used for detecting the cytosine methylation state of genomic DNA, by analysis of said sequence or treated versions of said sequence (according to SEQ ID NO. 1 through SEQ ID NO. 100 and sequences complementary thereto).

These probes enable improved detection, diagnosis, treatment and monitoring of breast cell proliferative disorders.

The set of oligonuleotides may also be used for detecting single nucleotide polymorphisms (SNPs) by analysis of said sequence or treated versions of said sequence according to one of SEQ ID NO. 1 through SEQ ID NO. 100.

According to the present invention, it is preferred that an arrangement of different oligonucleotides and/or PNA-oligomers (a so-called "array") made available by the present invention is present in a manner that it is likewise bound to a solid phase.

This array of different oligonucleotide- and/or PNA-oligomer sequences can be characterized in that it is arranged on the solid phase in the form of a rectangular or hexagonal lattice. The solid phase surface is preferably composed of silicon, glass, polystyrene, aluminum, steel, iron, copper, nickel, silver, or gold. However, nitrocellulose and plastics such as nylon, which can exist in the form of pellets or also as resin matrices, are suitable alternatives.

Therefore, a further subject matter of the present disclosure is a method for manufacturing an array fixed to a carrier material for the improved detection, diagnosis, treatment and monitoring of breast cell proliferative disorders and/or detection of the predisposition to breast cell proliferative disorders. In said method, at least one oligonucleotide according to the present invention is coupled to a solid phase. Methods for manufacturing such arrays are known, for example, from U.S. Pat. No. 5,744,305 by means of solid-phase chemistry and photolabile protecting groups. A further subject matter of the present invention relates to a DNA chip for the improved detection, diagnosis, treatment and monitoring of breast cell proliferative disorders. Furthermore, the DNA chip enables detection of the predisposition to breast cell proliferative disorders.

The DNA chip contains at least one nucleic acid and/or oligonucleotide according to the present invention. DNA-chips are known, for example, in U.S. Pat. No. 5,837,832.

The invention also relates to a composition or array comprising nucleic acids with sequences which are identical to at least 10 of the sequences according to SEQ ID NO. 1 to SEQ ID NO. 100, wherein the composition or array comprises no more than 100 different nucleic acid molecules.

The present disclosure relates to a composition or array comprising at least 5 sequences with a cumulative p-value of under 0.001, preferred under 0.0001.

Moreover, a subject matter of the present disclosure is a kit which may be composed, for example, of a bisulfite containing reagent, a set of primer oligonucleotides containing at least two oligonucleotides whose sequences in each case correspond to or are complementary to an at least 15 base long segment of the base sequences specified in SEQ ID NO. 1 to SEQ ID NO. 100. It is preferred that the primers are for SEQ ID NO. 1 through SEQ ID NO. 10 and SEQ ID NO. 50 through SEQ ID NO. 60.

### EXAMPLES

### SAMPLES

Patient samples were obtained from Norwegian Radium Hospital, Oslo, Norway and the National Cancer Institute's Cooperative Human Tissue Network (CHTN), and patient consent obtained as per legal requirements.

### CPG ISLANDS

Annotated CpG islands were obtained from the UCSC genome browser. These islands were predicted using the published Gardiner-Garden definition ( Gardiner-Garden, M. and M. Frommer (1987). "CpG islands in vertebrate genomes." J Mol Biol 196(2): 261-82) involving the following criteria: length >= 200bp, %GC >= 50%, observed/expected CpG >= 0.6. There are ~26219 CpG islands in the range of 200bp to 2000bp in the genome. These islands are well covered by Msp I restriction fragmentation.

Arrays were manufactured by Nimblegen Systems Inc using the 390K format to the following specifications. The CpG island annotation from human genome build 33 (hg17) was used to design a 50 mer tiling array. The 50 mers were shifted on either side of the island sequence coordinates to evenly distribute the island. The 390K format has 367,658 available features which would not fit all islands with a 50 mer tiling. Therefore we made a cutoff on the islands to be represented based on size, with only CpG islands of size 200b-2000b being assayed. Control probes were designed to represent background signal. Sample preparation: representations, has been described previously (Lucito, R., J. Healy, et al. (2003). "Representational oligonucleotide microarray analysis: a high-resolution method to detect genome copy number variation" Genome Res 13(10): 2299-305.), with the following changes. The primary restriction endonuclease used is MspI. After the digestion the following linkers were ligated (MspI24mer, and MSPI12mer). The 12 mer is not phosphorylated and does not ligate. After ligation the material is cleaned by phenol chloroform, precipitated, centrifuged, and re-suspended. The material is divided in two, half being digested by the endonuclease McrBC and the other half being mock digested. As few as four 250µl tubes were used for each sample pair for amplification of the representation each with a 100ul volume reaction. The cycle conditions were 95°C for 1 min, 72°C for 3 min, for 15 cycles, followed by a 10-min extension at 72°C. The contents of the tubes for each pair were pooled when completed. Representations were cleaned by phenol:chloroform extraction, precipitated, resuspended, and the concentration determined. DNA was labeled as described with minor changes (Lucito, R., J. Healy, et al. (2003). "Representational oligonucleotide microarray analysis: a high-resolution method to detect genome copy number variation" Genome Res 13(10): 2291-305.). Briefly, 2 µg of DNA template was placed (dissolved in TE at pH 8) in a 0.2 ml PCR tube. 5 µl of random nonamers (Sigma Genosys) were added brought up to 25 µl with dH₂O, and mixed. The tubes were placed in Tetrad at 100°C for 5 min, then on ice for 5 min. To this 5 µl of NEB Buffer 2, 5 µl of dNTPs (0.6 nM dCTP, 1.2 nM dATP, dTTP, dGTP), 5 µl of label (Cy3-dCTP or Cy5-dCTP) from GE Healthcare, 2 µl of NEB Klenow fragment, and 2 µl dH₂O was added. Procedures for hybridization and washing were followed as reported previously (Lucito, R., J. Healy, et al. (2003). "Representational oligonucleotide microarray analysis: a high-resolution method to detect genome copy number variation" Genome Res 13(10): 2291-305) with the exception that oven temperature for hybridization was increased to 50°C. Arrays were scanned with an Axon GenePix 4000B scanner set at a pixel size of 5 µm. GenePix Pro 4.0 software was used to quantify the intensity for the arrays. Array data were imported into S-PLUS for further analysis.

### DATA ANALYSIS

Microarray images were scanned on GenePix 4000B scanner and data extracted using Nimblescan software (Nimblegen Systems Inc). For each probe, the geometric mean of the ratios (GeoMeanRatio) of McrBc and control treated samples were calculated for each experiment and its associated dye swap. The GeoMeanRatios of all the samples in a dataset were then normalized using quantile normalization method (Bolstad, B. M., R. A. Irizarry, et al. (2003). "A comparison of normalization methods for high density oligonucleotide array data based on variance and bias" Bioinformatics 19(2): 185-93). The normalized ratios for each experiment were then collapsed to get one value for all probes in every MspI fragment using a median polish model. The collapsed data was then used for further analysis.

Analysis of variance was used to identify the most significant islands. In order to determine the most consistently occurring changes in methylation between tumor and normal samples, we used a t-test approach. Using a p-value cutoff of 0.001 after correction for multiple testing (False Discovery Rate, Benjamini and Hotchberg (Benjamini 1995)), we obtained a list of 916 MspI fragments that show differential methylation, derived from these 916 fragments based on the association with genes.

Supervised learning: We used a supervised machine learning classifier to identify the number of features required to differentiate tumor samples from normal. A publicly available support vector machine (SVM) library (LibSVM Ver 2.8) was used to obtain classification accuracy using a leave one out method (Lin, C.J. and Chang C.C. (2001). LIBSVM: a library for support vector machines). The methylation features for classification were first selected using t-test among the training data alone. The SVM was then trained on the top 10, 50 and 100 features using the radial basis function (RBF) kernel.

For N samples, t-tests were performed for (N-1) samples to identify fragments with significant differences in methylation ratios. For the breast dataset this was performed 52 times for all 52 breast samples, so that each sample is left out once during the t-test calculations. The methylation ratios of top 10 fragment features from (N-1) samples were then used for training the SVM and the ratios from one untrained sample was used for testing. Based on just 10 features, we can arrive at a classification accuracy of 94% (Total correct predictions/total predictions, 49/52). The sensitivity for Tumor detection was 92.5% (37/40), the specificity for tumor detection was 100%). Increasing the feature size to 50 gives a classification rate of 96% (50/52 classified correctly). Interestingly, the two tumor samples that were classified as normal in this analysis were also the closest to normal in both gene expression and ROMA analysis.

### DETECTION OF METHYLTED SITES

In a preferred embodiment, the method comprises the following steps: In the first step of the method the genomic DNA sample must be isolated from sources such as cell lines, tissue or blood samples. DNA extraction may be performed by means that are standard to one skilled in the art. These include the use of detergent lysates, sonification and vortexing with glass beads. Once the nucleic acids have been extracted the genomic double stranded DNA is used in the analysis.

In a preferred embodiment, the DNA my be cleaved prior to the next step of the method, this may by any means standard in the state of the art, in particular, but not limited to, with restriction endonucleases.

In the second step of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pretreatment' hereinafter.

The above described treatment of genomic DNA is preferably carried out with bisulfite (sulfite, disulfite) and subsequent alkaline hydrolysis which results in a conversion of non-methylated cytosine nucleobases to uracil or to another base which is dissimilar to cytosine in terms of base vairine behavior. If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The converted DNA is then used for the detection of methylated cytosines.

Fragments are amplified. Because of statistical and practical considerations, preferably more than ten different fragments having a length of 100-2000 base pairs are amplified. The amplification of several DNA segments can be carried out simultaneously in one and the same reaction vessel. Usually, the amplification is carried out by means of a polymerase chain reaction (PCR). The design of such primers is obvious to one skilled in the art. These should include at least two oligonucleotides whose sequences are each reverse complementary or identical to an at least 15 base-pair long segment of the base sequences specified in the appendix (SEQ ID NO. 1 through SEQ ID NO. 100). Said primer oligonucleotides are preferably characterized in that they do not contain any CpG dinucleotides. In a particularly preferred embodiment of the method, the sequence of said primer oligonucleotides are designed so as to selectively anneal to and amplify, only the breast cell specific DNA of interest, thereby minimizing the amplification of background or non relevant DNA. In the context of the present invention, background DNA is taken to mean genomic DNA which does not have a relevant tissue specific methylation pattern, in this case, the relevant tissue being breast cells, both healthy and diseased.

According to the present invention, it is preferred that at least one primer oligonucleotide is bound to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminum, steel, iron, copper, nickel, silver, or gold. However, it is possible for other materials such as nitrocellulose or plastics to be used as well. The fragments obtained by means of the amplification may carry a directly or indirectly detectable label. Preferred are labels in the form of fluorescence labels, radionuclides, or detachable molecule fragments having a typical mass which can be detected in a mass spectrometer. Preferably, the fragments that are produced have a single positive or negative net charge for better detectability in the mass spectrometer. The detection may be carried out and visualized by means of matrix assisted laser desorption/ionization mass spectrometry (MALDI) or by using electron spray mass spectrometry (ESI).

In the next step, the nucleic acid amplicons are analyzed in order to determine the methylation status of the genomic DNA prior to treatment.

The post treatment analysis of the nucleic acids may be carried out using alternative methods. Several methods for the methylation status specific analysis of the treated nucleic acids are known, other alternative methods will be obvious to one skilled in the art.

Using several methods known in the art the analysis may be carried out during the amplification step of the method. In one such embodiment, the methylation status of preselected CpG positions within the nucleic acids comprising SEQ ID NO. 1 through SEQ ID NO. 100 may be detected by use of methylation specific primer oligonucleotides. This technique has been described in U.S. Pat. No. 6,265,171.

## Claims

1. A method for the analysis of breast cancer disorders, comprising:
determining the genomic methylation status of one or more CpG dinucleotides in each sequence of the group of sequences of SEQ ID NO. 1 to SEQ ID NO. 10.

2. A method for the analysis of breast cancer disorders, comprising:
determining the genomic methylation status of one or more CpG dinucleotides in each sequence of the group of sequences of SEQ ID NO. 51 to SEQ ID NO. 60.

3. The method according to claim 1 or 2 for the detection of breast cancer in a subject, wherein said method is to be carried out on a sample obtained from a subject to be analyzed.

4. The method according to any one of claims 1 to 3, wherein said method further comprises:
determining the genomic methylation status of one or more CpG dinucleotides in one or more of the DNA sequences selected from the group of sequences according to SEQ ID NO. 11 to SEQ ID NO. 49 and SEQ ID NO. 61 to SEQ ID NO. 100.

5. The method according to any one of claims 1 to 4, wherein the genomic methylation status is determined for the panel of sequences according to SEQ ID NO. 1 to SEQ ID NO. 10 and SEQ ID NO. 51 to SEQ ID NO. 60.

6. The method according to any one of claims 1 to 5, further comprising:
(a) imputing the one or more results from the methylation status test into a classifier that is obtained from a Diagnostic Multi Variate Model;
(b) calculating a likelihood as to whether the sample is from a normal tissue or an breast cancer tissue; and
(c) calculating an associated p-value for the confidence in the prediction.

7. The method according to any one of claims 1 to 6, wherein the genomic methylation status is determined by means of one or more of the methods selected form the group of:
(a) bisulfite sequencing;
(b) pyrosequencing;
(c) methylation-sensitive single-strand conformation analysis (MS-SSCA);
(d) high resolution melting analysis (HRM);
(e) methylation-sensitive single nucleotide primer extension (MS-SnuPE);
(f) base-specific cleavage/MALDI-TOF;
(g) methylation-specific PCR (MSP);
(h) microarray-based methods; and
(i) Msp I cleavage.

8. The method according to any one of claims 3 to 7, wherein the sample to be analyzed is from a tissue type selected from the group of tissues such as a tissue biopsy from the tissue to be analyzed, vaginal tissue, tongue, pancreas, liver, spleen, ovary, muscle, joint tissue, neural tissue, gastrointestinal tissue, tumor tissue, body fluids, blood, serum, saliva, and urine.

9. The method according to any of claims 3 to 8, wherein the breast cancer to be detected is a primary cancer.

10. A set of probe molecules for the detection of the cytosine methylation state of genomic DNA according to SEQ ID NO. 1 to SEQ ID NO. 10 or sequences complementary thereto, wherein the set comprises probe molecules specific for the DNA sequence of SEQ ID NO. 1 to SEQ ID NO. 10.

11. A composition or an array comprising nucleic acid molecules with sequences which are identical to at least 10 of the sequences according to SEQ ID NO. 1 to SEQ ID NO. 100, wherein the composition or array comprises no more than 100 different nucleic acid molecules.

## Patentansprüche

1. Verfahren zur Analyse von Brustkrebserkrankungen, das Folgendes umfasst:
Ermitteln des genomischen Methylierungsstatus von einem oder mehreren der CpG-Dinukleotide in jeder Sequenz der Gruppe von Sequenzen von SEQ ID NO. 1 bis SEQ ID NO. 10.

2. Verfahren zur Analyse von Brustkrebserkrankungen, das Folgendes umfasst:
Ermitteln des genomischen Methylierungsstatus von einem oder mehreren der CpG-Dinukleotide in jeder Sequenz der Gruppe von Sequenzen von SEG ID NO. 51 bis SEQ ID NO. 60.

3. Verfahren nach Anspruch 1 oder 2 zum Erkennen von Brustkrebs in einer Person, wobei das genannte Verfahren an einer von der Person gewonnenen zu analysierenden Probe durchzuführen ist.

4. Verfahren nach einem der Ansprüche 1 bis 3, wobei das genannte Verfahren weiterhin Folgendes umfasst:
Ermitteln des genomischen Methylierungsstatus von einem oder mehreren der CpG-Dinukleotiden in einer oder mehreren der DNA-Sequenzen ausgewählt aus der Gruppe von Sequenzen gemäß SEQ ID NO. 11 bis SEQ ID NO. 49 und SEQ ID NO. 61 bis SEQ ID NO. 100.

5. Verfahren nach einem der Ansprüche 1 bis 4, wobei der genomische Methylierungsstatus für das Panel von Sequenzen gemäß SEQ ID NO. 1 bis SEQ ID NO.10 und SEQ ID NO. 51 bis SEQ ID NO. 60 ermittelt wird.

6. Verfahren nach einem der Ansprüche 1 bis 5, das weiterhin Folgendes umfasst:
(a) Eingeben von einem oder mehreren Ergebnis aus der Prüfung des Methylierungsstatus in einen Klassifizierer, der anhand eines diagnostischen Multivariate-Modells erlangt wird;
(b) Berechnen einer Wahrscheinlichkeit in Bezug darauf, ob die Probe von einem normalen Gewebe oder einem Brustkrebsgewebe stammt; und
(c) Berechnen eines zugehörigen p-Wertes für den Konfidenzwert der Vorhersage.

7. Verfahren nach einem der Ansprüche 1 bis 6, wobei der genomische Methylierungsstatus mit Hilfe einem oder mehreren der Verfahren ermittelt wird, die aus folgender Gruppe ausgewählt werden:
(a) Bisulfit-Sequenzierung;
(b) Pyrosequenzierung;
(c) Methylierungssensitive Einzelstrang-Konformationsanalyse (MS-SSCA);
(d) hochauflösende Schmelzanalyse (HRM);
(e) Methylierungsssensitive Einzel-Nukleotid-Primer-Extension (SM-SnuPE);
(f) Basenspezifische Spaltung/MALDI-TOF;
(g) Methylierungsspezifische PCR (MSP);
(h) mikroarray-basierende Verfahren; und
(i) Msp II Spaltung.

8. Verfahren nach einem der Ansprüche 3 bis 7, wobei die zu analysierende Probe von einem Gewebetyp ist, der ausgewählt wird aus der Gruppe von Geweben wie einer Gewebebiopsie des zu analysierenden Gewebes, Vaginalgewebe, Zunge, Pankreas, Leber, Milz, Eierstöcke, Muskeln, Gelenkgewebe, neurales Gewebe, gastrointestinales Gewebe, Tumorgewebe, Körperflüssigkeiten, Blut, Serum, Speichel und Urin.

9. Verfahren nach einem der Ansprüche 3 bis 8, wobei der zu erkennende Brustkrebs ein Primärtumor ist.

10. Reihe von Sondenmolekülen zur Detektion des Cytosin-Methylierungsstatus der genomischen DNA gemäß SEQ ID NO. 1 bis SEQ ID NO. 10 oder hierzu komplementären Sequenzen, wobei die Reihe Sondenmoleküle umfasst, die für die DNA-Sequenz von SEQ ID NO. 1 bis SEQ ID NO. 10 spezifisch sind.

11. Zusammensetzung oder Array mit Nukleinsäuremolekülen mit Sequenzen, die mit mindestens 10 der Sequenzen gemäß SEQ ID NO. 1 bis SEQ ID NO. 100 identisch sind, wobei die Zusammensetzung oder das Array nicht mehr als 100 verschiedene Nukleinsäuremoleküle umfasst.

## Revendications

1. Procédé pour l'analyse de troubles du cancer du sein, comprenant :
la détermination de l'état de méthylation génomique d'un ou plusieurs dinucléotides CpG dans chaque séquence du groupe de séquences de la SEQ.ID N°1 à la SEQ. ID N°10.

2. Procédé pour l'analyse de troubles du cancer du sein, comprenant :
la détermination de l'état de méthylation génomique d'un ou plusieurs dinucléotides CpG dans chaque séquence du groupe de séquences de la SEQ. ID N°51 à la SEQ. ID N°60.

3. Procédé selon la revendication 1 ou 2 pour la détection d'un cancer du sein chez un sujet, dans lequel ledit procédé doit être réalisé sur un échantillon obtenu sur un sujet à analyser.

4. Procédé selon l'une quelconque des revendications 1 à 3, dans lequel ledit procédé comprend :
la détermination de l'état de méthylation génomique d'un ou plusieurs dinucléotides CpG dans une ou plusieurs des séquences d'ADN choisies dans le groupe de séquences selon la SEQ. ID N°11 à la SEQ. ID N°49 et selon la SEQ. ID N°61 à la SEQ. ID N°100.

5. Procédé selon l'une quelconque des revendications 1 à 4, dans lequel l'état de méthylation génomique est déterminé pour le panel de séquences selon les SEQ. ID N°1 à SEQ. ID N°10 et SEQ. ID N°51 à SEQ. ID N°60.

6. Procédé selon l'une quelconque des revendications 1 à 5, comprenant en outre :
(a) l'imputation du ou des résultats provenant du test d'état de méthylation dans un classificateur, obtenu à partir d'un Modèle Multivariables de diagnostic ;
(b) le calcul d'une probabilité quant au fait que l'échantillon provienne d'un tissu normal ou d'un tissu de cancer du sein ; et
(c) le calcul d'une valeur p associée pour la confiance dans la prédiction.

7. Procédé selon l'une quelconque des revendications 1 à 6, dans lequel l'état de méthylation génomique est déterminé au moyen d'un ou plusieurs des procédés choisis parmi le groupe constitué de :
(a) séquençage de bisulfite
(b) pyro-séquençage
(c) analyse de conformation simple brin sensible à la méthylation (MS-SSCA)
(d) analyse de fusion haute résolution (HRM) ;
(e) extension d'amorce nucléotidique unique sensible à la méthylation (MS-SnuPE) ;
(f) clivage spécifique à la base/MALDI-TOF ;
(g) PCR spécifique à la méthylation (MSP) ;
(h) procédés à base de biopuce ; et
(i) clivage Msp I.

8. Procédé selon l'une quelconque des revendications 3 à 7, dans lequel l'échantillon à analyser provient d'un type de tissu choisi dans le groupe de tissus, comme une biopsie de tissu à partir du tissu à analyser, le tissu vaginal, la langue, le pancréas, le foie, la rate, les ovaires, le muscle, le cartilage des articulations, le tissu nerveux, le tissu gastro-intestinal, le tissu tumoral, les fluides corporels, le sang, le sérum, la salive et l'urine.

9. Procédé selon l'une quelconque des revendications 3 à 8, dans lequel le cancer du sein à détecter est un cancer primaire.

10. Ensemble de molécules sondes pour la détection de l'état de méthylation de cytosine de l'ADN génomique selon la SEQ. ID N°1 à la SEQ. ID N°10 ou des séquences complémentaires, dans lequel l'ensemble comprend des molécules sondes spécifiques pour la séquence d'ADN de la SEQ. ID N°1 à la SEQ. ID N°10.

11. Composition ou série comprenant des molécules d'acide nucléique avec des séquences qui sont identiques à au moins 10 des séquences selon la SEQ. ID N°1 à SEQ. ID N°100, dans laquelle la composition ou série comprend au plus 100 molécules d'acide nucléique différentes.
